(19) **Europäisches Patentamt European Patent Office Office européen des brevets**

(11) **EP 4 681 725 A1**

(12) **EUROPEAN PATENT APPLICATION**
published in accordance with Art. 153(4) EPC

(43) Date of publication:
**21.01.2026 Bulletin 2026/04**

(21) Application number: **24774877.5**

(22) Date of filing: **15.03.2024**

(51) International Patent Classification (IPC):
*A61K 38/20* (2006.01)    *A61P 9/00* (2006.01)
*C07K 14/54* (2006.01)    *C12N 5/0735* (2010.01)
*C12N 5/077* (2010.01)    *C12Q 1/02* (2006.01)
*G01N 33/15* (2006.01)    *G01N 33/50* (2006.01)
*G01N 33/68* (2006.01)

(52) Cooperative Patent Classification (CPC):
**A61K 38/20; A61P 9/00; C07K 14/54; C12N 5/06; C12Q 1/02; G01N 33/15; G01N 33/50; G01N 33/68**

(86) International application number:
**PCT/JP2024/010348**

(87) International publication number:
**WO 2024/195741 (26.09.2024 Gazette 2024/39)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **17.03.2023 JP 2023042924**

(71) Applicant: **Tokai University Educational System Tokyo, 151-0063 (JP)**

(72) Inventors:
• **HAYASHI, Takeharu**
  **Isehara-shi, Kanagawa 259-1193 (JP)**

• **KIMURA, Akinori**
  **Tokyo 113-8510 (JP)**
• **TANIMOTO, Kousuke**
  **Tokyo 113-8510 (JP)**

(74) Representative: **Isarpatent**
  **Patent- und Rechtsanwälte Partg mbB**
  **Friedrichstraße 31**
  **80801 München (DE)**

Remarks:
The complete document including Reference Table(s) and the Sequence Listing(s) can be downloaded from the EPO website

(54) **THERAPEUTIC AGENT AND EXAMINATION METHOD FOR CARDIAC HYPERTROPHY**

(57)    An object to be achieved by the present invention is to identify a gene involved in cardiac hypertrophy, and provide a therapeutic agent and an examination method for cardiac hypertrophy each targeting the gene. The present invention provides a composition for suppressing cardiac hypertrophy, including interleukin-17D as an active ingredient.

Fig. 2A

EP 4 681 725 A1

**Description**

Technical Field

The present invention relates to a therapeutic agent and an examination method for cardiac hypertrophy, and more particularly, to a composition for suppressing cardiac hypertrophy including interleukin-17D (IL-17D) as an active ingredient and an examination method for cardiac hypertrophy including detecting IL-17D. The present invention also relates to a screening method for a substance having activity to suppress cardiac hypertrophy with use of a cardiomyocyte or a non-human animal having a suppressed function of IL-17D. The present invention also relates to a production method for a differentiated cardiomyocyte with use of IL-17D.

Background Art

Cardiac failure is one of the primary causes of morbidity and mortality of cardiovascular diseases in the world, and cardiomyopathy is one of the main causes thereof. Hypertrophic cardiomyopathy (HCM) is a primary myocardial disease characterized by hypertrophy and diastolic dysfunction of the left ventricle. HCM occurs most frequently among hereditary cardiovascular diseases, and is the main cause of cardiac sudden deaths in youths, and it is reported that 0.2% of the general population has HCM.

HCM was first reported to be mainly caused by pathologic mutations in genes encoding structural proteins of sarcomere, but those mutations have been observed only in about half of HCM patients (Non-patent Literature 1). Recent studies have reported other mutations of genes encoding calcium-related proteins and hypertrophic signal-related proteins, and shown the presence of various causes of HCM (Non-patent Literatures 2 and 3). Further, there has been still no effective therapy for improving a prognosis of HCM.

Citation List

Non-patent Literature

NPL 1: B.J. Maron et al., Journal of the American College of Cardiology, 2012, Vol. 60, pp. 705-715
NPL 2: A.J. Marian et al., Circ Res, 2017, Vol. 121, pp. 749-770
NPL 3: C.E. Seidman et al., Circ Res, 2011, Vol. 108, pp. 743-750
NPL 4: R. Saddawi-Konefka et al., Cell Rep, 2016, Vol. 16, pp. 2348-2358
NPL 5: R. Seelige et al., Cytokine, 2017, Vol. 91, pp. 10-12
NPL 6: T. Starnes et al., J Immunol, 2002, Vol. 169, pp. 642-646
NPL 7: Timothy O'Sullivan et al., Cell Rep, 2014, Vol. 7, No. 4, pp. 989-998

Summary of Invention

Technical Problem

The present invention has been made in consideration of the above-mentioned problems of the related art, and identifies a gene involved in cardiac hypertrophy such as HCM. In addition, an object of the present invention is to provide a therapeutic agent and an examination method for cardiac hypertrophy each targeting the gene.

Solution to Problem

To solve the problems, the inventors of the present invention first performed exome analysis for families having a plurality of HCM patients and thus identified a read-through mutation in IL17D gene in all HCM patients in the same pedigree.

IL-17D is a cytokine belonging to the IL-17 family. Among the IL-17 family consisting of IL-17A to IL-17F, IL-17A is known as a cytokine involved in inflammation, bacterial infection, and an autoimmune disease. In contrast, it is known that IL-17D increases with some kinds of tumors or virus infections, and that treatment of cultured endothelial cells with IL-17D leads to increased IL-6 and IL-8, but little has been known about a function of IL-17D. In addition, few researches have been reported for myocardia (Non-patent Literatures 4 to 7).

Further, comparison of homologies between IL-17D and other members of the IL-17 family with Uniprot (https://www.uniprot.org) revealed only 20.5% homology with IL-17A and low homology up to 25.9% with IL-17B, suggesting that IL-17D has a distinct function as compared to other members of the IL-17 family.

Thus, on the basis of rare IL17D variants observed in HCM, the inventors of the present invention hypothesized that IL-17D could have a function to suppress cardiac hypertrophy. Then, to verify the hypothesis, cultured cardiomyocytes pretreated with a drug for inducing cardiac hypertrophy were treated with IL-17D, thereby revealing that the expression levels of cardiac hypertrophy markers were decreased in the cells.

Meanwhile, it was found that cultured cardiomyocytes subjected to genome editing of IL17D had increased expression levels of cardiac hypertrophy markers, and further, that a mouse having a read-through mutation in IL17D displayed cardiac hypertrophy. In addition, the inventors of the present invention elucidated that such increased marker and cardiac hypertrophy were alleviated by treatment with IL-17D.

Further, the inventors of the present invention also revealed that treatment with IL-17D promoted differentiation of normal human iPS cell-derived cardiomyocytes.

As described above, the inventors have found that IL-17D is a cardiac hypertrophy suppressor and has a function to promote differentiation of myocardia, and completed the present invention. That is, the present invention provides the following aspects.

[1] A composition for suppressing cardiac hypertrophy and/or diastolic dysfunction, including interleukin-17D as an active ingredient.

[2] The pharmaceutical composition according to Item [1], wherein the pharmaceutical composition is a pharmaceutical composition for treating or preventing a disease associated with cardiac hypertrophy and/or diastolic dysfunction.

The present invention also provides: a use of interleukin-17D for producing the composition for suppressing cardiac hypertrophy and/or diastolic dysfunction; a use of interleukin-17D for suppressing cardiac hypertrophy and/or diastolic dysfunction, or treating or preventing a disease associated with cardiac hypertrophy and/or diastolic dysfunction; interleukin-17D for use in suppressing cardiac hypertrophy and/or diastolic dysfunction, or treating or preventing a disease associated with cardiac hypertrophy and/or diastolic dysfunction; a method of treating or preventing a disease associated with cardiac hypertrophy and/or diastolic dysfunction, including administering an effective amount of interleukin-17D to a subject in need thereof; and a suppressant for cardiac hypertrophy and/or diastolic dysfunction, including interleukin-17D.

Further, the present invention also provides the following aspects.

[3] An examination method for cardiac hypertrophy and/or diastolic dysfunction, including a step of detecting interleukin-17D for a sample isolated from a test subject.

[4] A screening method for a substance having activity to suppress cardiac hypertrophy and/or diastolic dysfunction, including the steps of: bringing a cardiomyocyte having a suppressed function of interleukin-17D into contact with a test substance; detecting expression of a cardiac hypertrophy marker and/or a diastolic dysfunction marker in the cardiomyocyte; and determining the test substance to have activity to suppress cardiac hypertrophy and/or diastolic dysfunction when the expression of the cardiac hypertrophy marker and/or the diastolic dysfunction marker detected in the detecting step is reduced as compared to no contact with the test substance.

[5] A screening method for a substance having activity to suppress cardiac hypertrophy and/or diastolic dysfunction, including the steps of: feeding a test substance to a non-human animal having a suppressed function of interleukin-17D; detecting cardiac hypertrophy and/or diastolic dysfunction in the non-human animal; and determining the test substance to have activity to suppress cardiac hypertrophy and/or diastolic dysfunction when cardiac hypertrophy and/or diastolic dysfunction detected in the detecting step is reduced as compared to no feeding of the test substance.

[6] A production method for a differentiated cardiomyocyte, including a step of culturing an immature cardiomyocyte in the presence of interleukin-17D.

[7] The production method according to Item [6], wherein the immature cardiomyocyte is an immature cardiomyocyte induced to differentiate from a pluripotent stem cell, or a cardiomyoblast.

Advantageous Effects of Invention

According to the present invention, the use of IL-17D allows suppression of cardiac hypertrophy, and in turn, treatment of a cardiac disease such as HCM. In addition, examination for cardiac hypertrophy can be performed by using IL-17D as an indicator.

Further, in the present invention, the use of cardiomyocytes or the like having a suppressed function of IL-17D enables screening for a substance having activity to suppress cardiac hypertrophy. In addition, the use of IL-17D also enables promotion of differentiation of immature cardiomyocytes.

Brief Description of Drawings

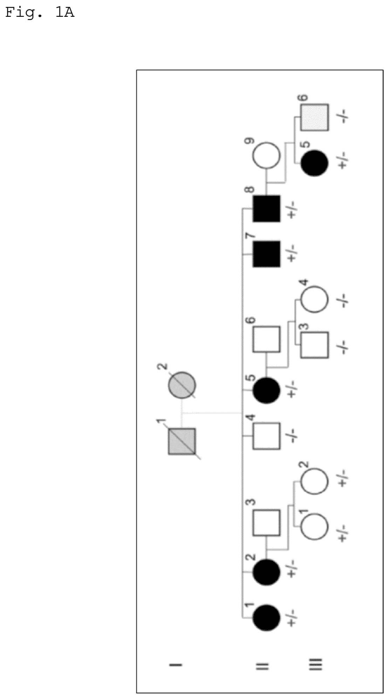

Fig. 1A is a diagram for illustrating a pedigree having onsets of HCM and their phenotypes and genotypes (squares: males, circles: females, black: HCM, gray (in colored presentation): unidentified phenotypes, white: no symptoms, +/-: cases with a heterozygous IL17D variant, -/-: cases with no IL17D variant).

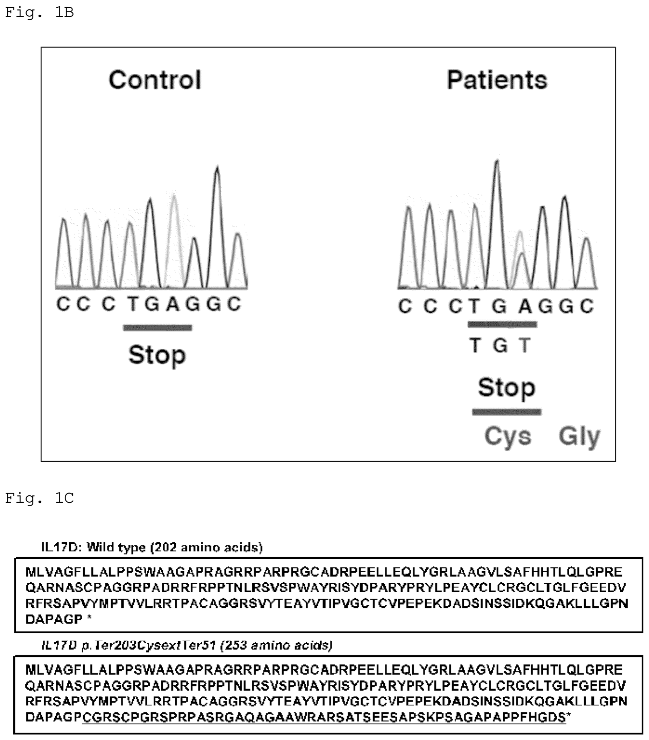

Fig. 1B is a diagram showing base sequences by Sanger method determined for IL17D genes of a control and HCM patients. The HCM patients have an IL17D (NM_138284.1): c.609 A>T, p.Ter203CysextTer51 heterozygous missense variant.

Fig. 1C is a diagram showing the amino acid sequence of wild-type IL-17D (upper panel, SEQ ID NO: 1) and the amino acid sequence of IL17D p.Ter203CysextTer51 present in familial HCM (lower panel, SEQ ID NO: 10), indicating that while wild-type IL-17D has 202 amino acids, IL17D p.Ter203CysextTer51 is predicted to have 253 amino acids.

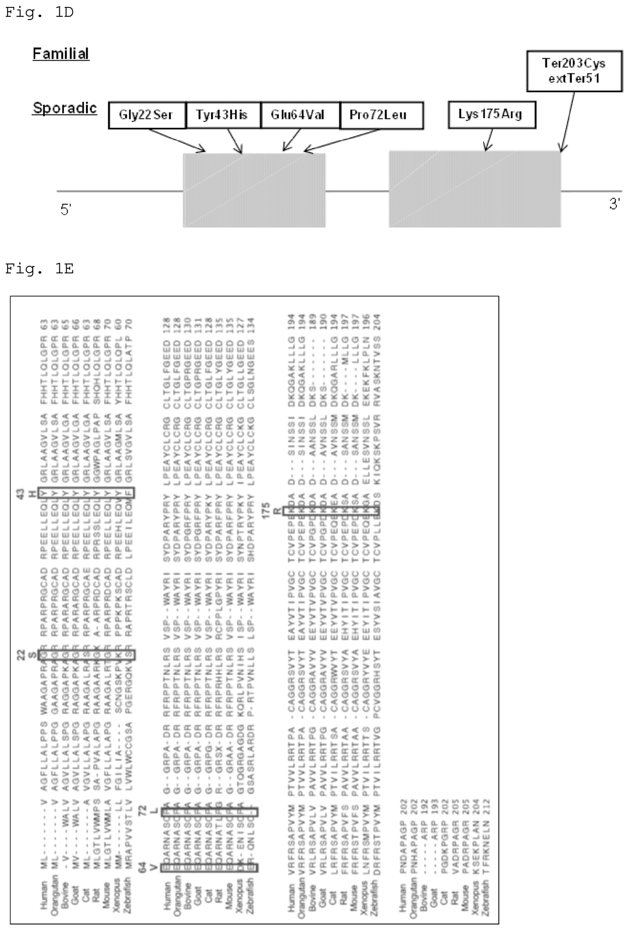

Fig. 1D is a schematic diagram for illustrating the exon/intron configuration of IL17D gene and positions of rare mutations found in HCM patients. The HCM patients with IL17D p.Ter203CysextTer51 have familial HCM, and the patients with p.Gly22Ser, p.Tyr43His, p.Glu64Val, p.Pro72Leu, or p.Lys175Arg have sporadic HCM.

Fig. 1E is a diagram showing alignment of IL-17D amino acid sequences of a human and other organism species. The sites enclosed by squares indicate positions of rare variants of IL17D identified in HCM. The human-derived sequence is set forth in SEQ ID NO: 1, the orangutan-derived sequence is set forth in SEQ ID NO: 2, the bovine-derived sequence is set forth in SEQ ID NO: 3, the goat-derived sequence is set forth in SEQ ID NO: 4, the cat-derived sequence is set forth in SEQ ID NO: 5, the rat-derived sequence is set forth in SEQ ID NO: 6, the mouse-derived sequence is set forth in SEQ ID NO: 7, the Xenopus-derived sequence is set forth in SEQ ID NO: 8, and the zebrafish-derived sequence is set forth in SEQ ID NO: 9.

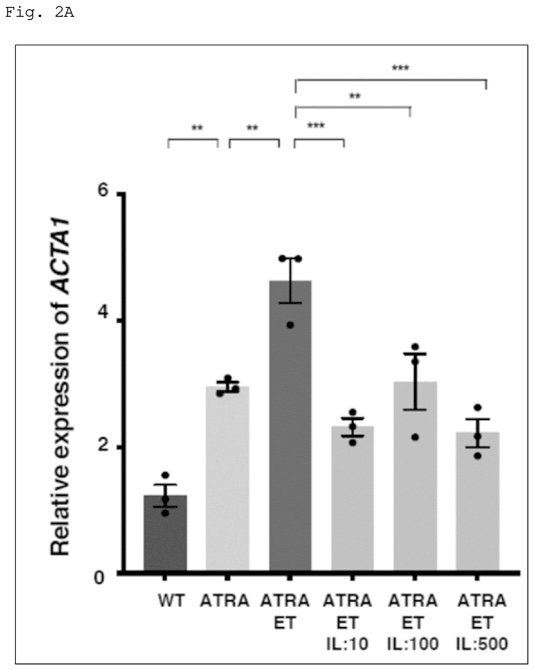

Fig. 2A is a graph showing relative gene expression of ACTA1 in each cell population. In Fig. 2A, normal H9c2 cells are indicated as "WT", and cells differentiated with all-trans retinoic acid (ATRA) are indicated as "ATRA". H9c2 cells differentiated with ATRA followed by induction of hypertrophy with 100 nM ET are indicated as "ATRA ET". Cells differentiated with ATRA, then hypertrophied with 100 nM ET, and administered 10 ng/mL, 100 ng/mL, or 500 ng/mL IL-17D are indicated as "ATRA ET IL:10", "ATRA ET IL:100", or "ATRA ET IL:500".

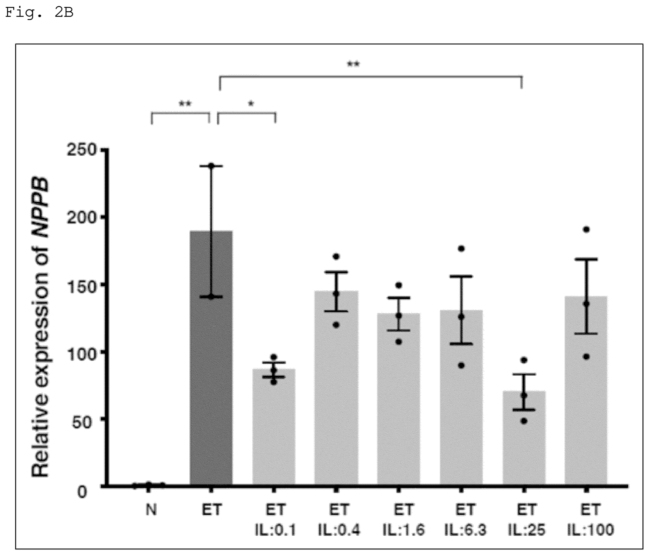

Fig. 2B is a graph showing relative expression of NPPB gene, which encodes BNP. In Fig. 2B, human iPS-derived cardiomyocytes (hiPS-CMs) are indicated as "N", and hiPS-CMs treated with 10 nM endothelin are indicated as "ET". Cells treated with 10 nM endothelin and then treated with 0.1 ng/mL to 100 ng/mL IL-17D are indicated as "ET IL:0.1" to "ET IL:100", respectively.

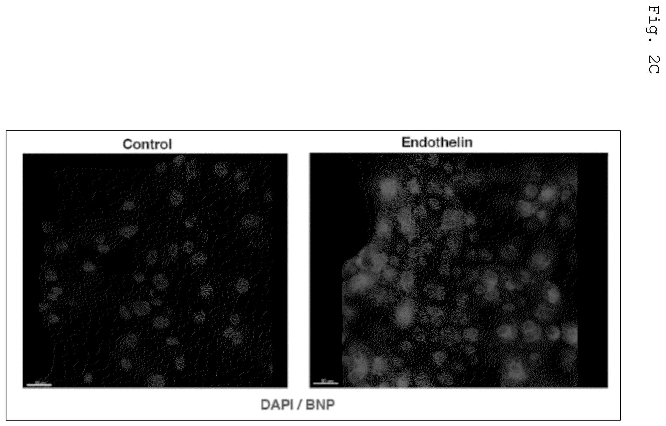

Fig. 2C represents images showing typical results of immunofluorescent staining analysis of endothelin-treated hiPS-CMs: right: showing a result of analysis for NT-proBNP expression in hiPS-CMs administered 10 nM endothelin (red in colored presentation), left: showing a result of a control under normal culture conditions. Nuclei are indicated by blue in colored presentation. Scale bar: 30 $\mu$m.

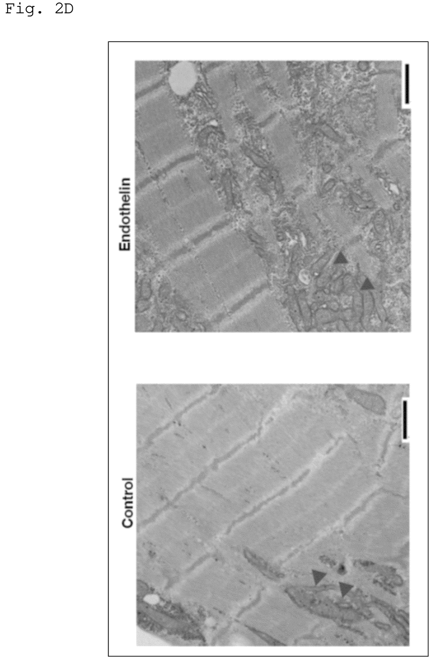

Fig. 2D represents images showing typical results of electron-microscopic observation of endothelin-treated hiPS-CMs: left: control, right: treated with 10 nM endothelin. Triangles point to mitochondria. Scale bar: 1 $\mu$m.

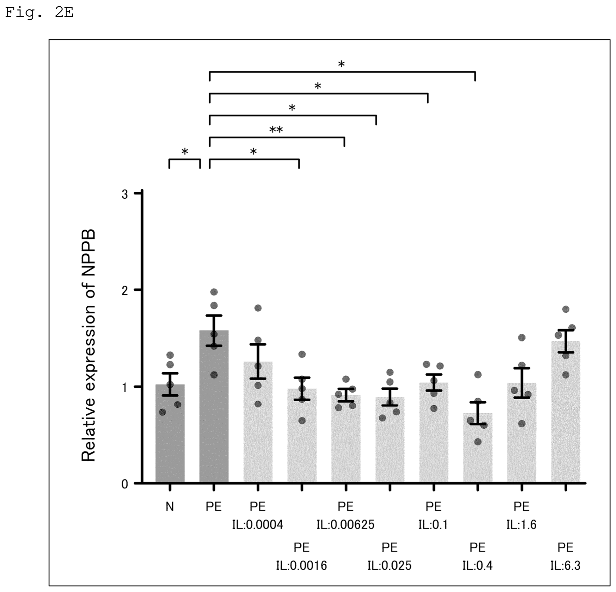

Fig. 2E is a graph showing the relative expression level of NPPB gene. In Fig. 2E, control hiPS-CMs are indicated as "N", and hiPS-CMs treated with 10 $\mu$M phenylephrine are indicated as "PE". hiPS-CMs treated with 10 $\mu$M phenylephrine and then treated with 0.0004 ng/mL to 6.3 ng/mL IL-17D are indicated as "PE IL:0.0004" to "PE IL:6.3", respectively.

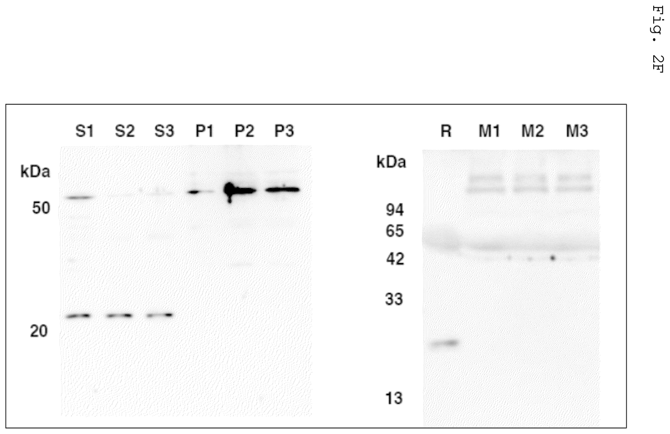

Fig. 2F represents images showing results of immunoblotting analysis of H9c2 cells stably expressing IL-17D. In Fig. 2F, on the left panel, "S1 to S3" represent cytoplasmic fractions, and "P1 to P3" represent membrane fractions. On the right panel, "M1 to M3" represent media after culturing of H9c2 cells, and "R" represents recombinant IL-17D.

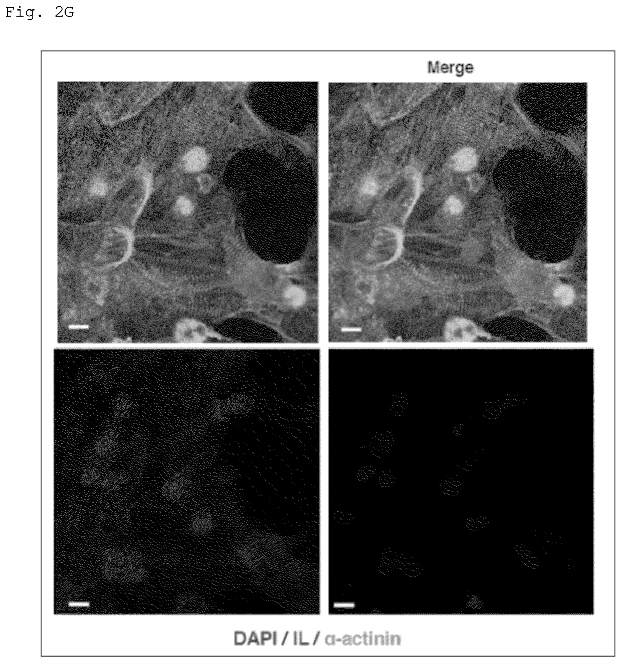

Fig. 2G represents images showing typical results of immunofluorescent staining analysis of hiPS-CMs. In Fig. 2G, expressions of $\alpha$-actinin, IL-17D, and nuclei are indicated by green, red, and blue in colored presentation, respectively. A merged image of those expressions is also shown on the upper right. Scale bar: 10 $\mu$m.

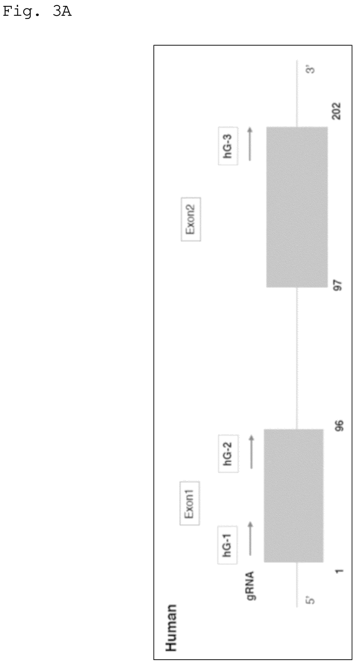

Fig. 3A is a schematic diagram for illustrating the exon/intron configuration of human IL17D gene and the sites of gRNAs designed for genome editing of hiPS-CMs.

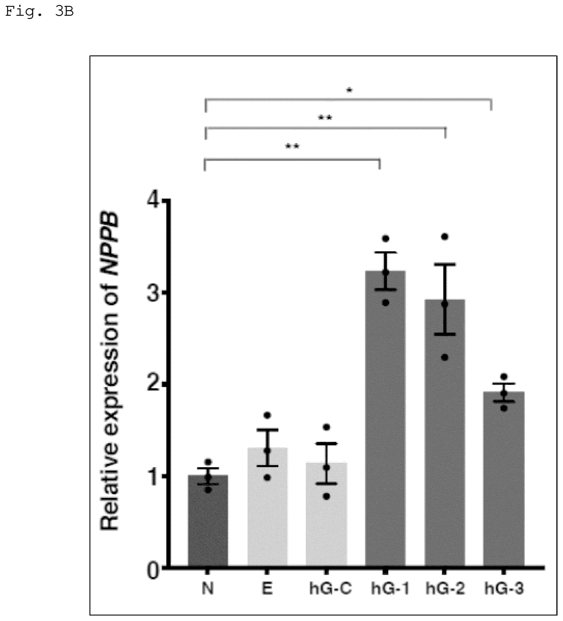

Fig. 3B is a graph showing results of subjecting IL17D gene to genome editing and analyzing relative expression of NPPB gene in hiPS-CMs. In Fig. 3B, a normal control is indicated as "N", cells treated only with a transfection enzyme are indicated as "E", cells transfected with a control vector are indicated as "hG-C", and cells transfected with the gRNA vector hG-1, hG-2, or hG-3 are indicated as "hG-1", "hG-2", or "hG-3".

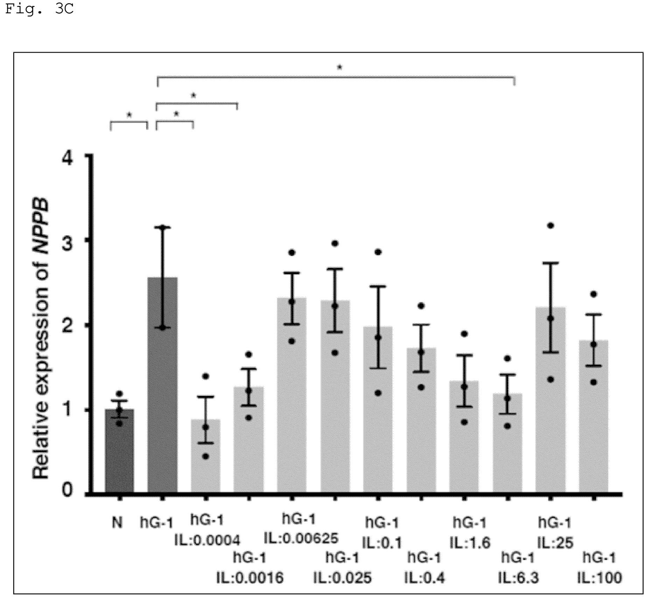

Fig. 3C is a graph showing results of subjecting IL17D gene to genome editing, administering IL-17D, and analyzing relative expression of NPPB gene in hiPS-CMs. In Fig. 3C, hiPS-CMs are indicated as "N", and cells subjected to genome editing for IL17D with hG-1 are indicated as "hG-1". In addition, cells subjected to the genome editing and then rescued with administration of 0.0004 ng/mL to 100 ng/mL IL-17D are indicated as "hG-1 IL:0.0004" to "hG-1 IL:100", respectively.

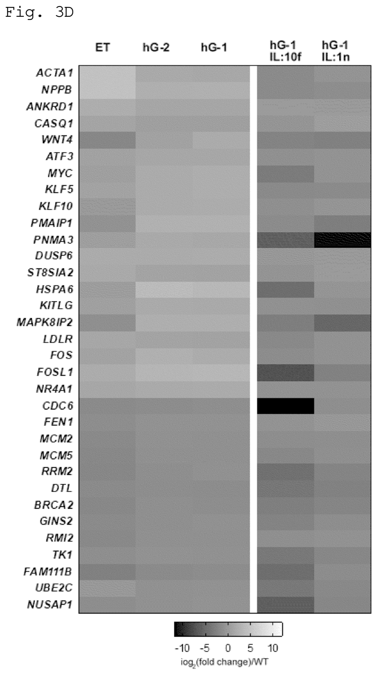

Fig. 3D is a heatmap showing expressions of typical genes identified by RNA-seq analysis of each cell population (the relative expression ratio of each gene in wild-type cells). hiPS-CMs treated with endothelin are indicated as "ET". hiPS-CMs subjected to genome editing with hG-2 or hG-1 are indicated as "hG-2" or "hG-1". hiPS-CMs subjected to genome editing with hG-1 and then administered 10 fg/mL or 1 ng/mL IL-17D are indicated as "hG-1 IL:10f" or "hG-1 IL:1n".

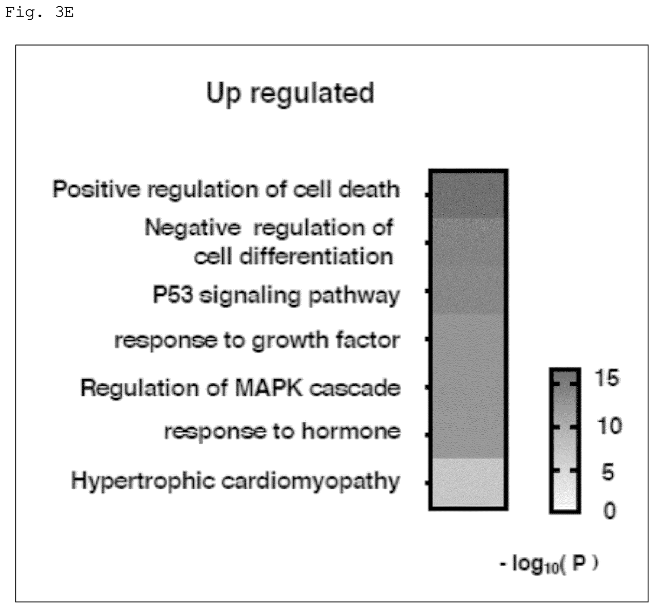

Fig. 3E is a heatmap showing results of Gene Ontology analysis of genes commonly upregulated between hiPS-CMs subjected to genome editing with hG-1 and hiPS-CMs subjected to genome editing with hG-2 in RNA-seq.

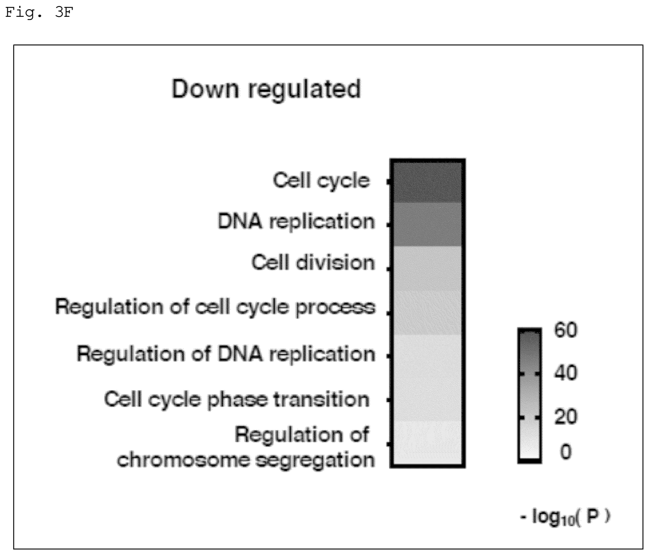

Fig. 3F is a heatmap showing results of Gene Ontology analysis of genes commonly downregulated between hiPS-CMs subjected to genome editing with hG-1 and hiPS-CMs subjected to genome editing with hG-2 in RNA-seq.

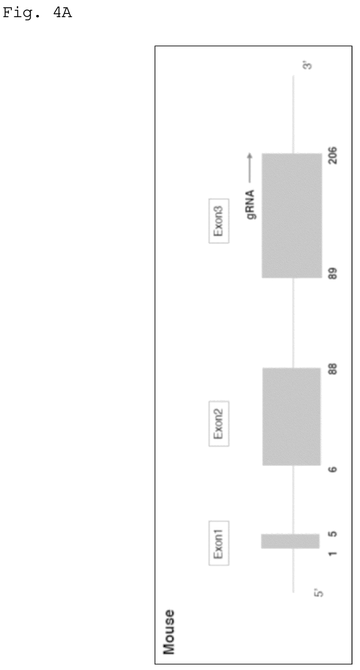

Fig. 4A is a schematic diagram for illustrating the exon/intron configuration of mouse IL17D gene and the site of a gRNA designed for genome editing.

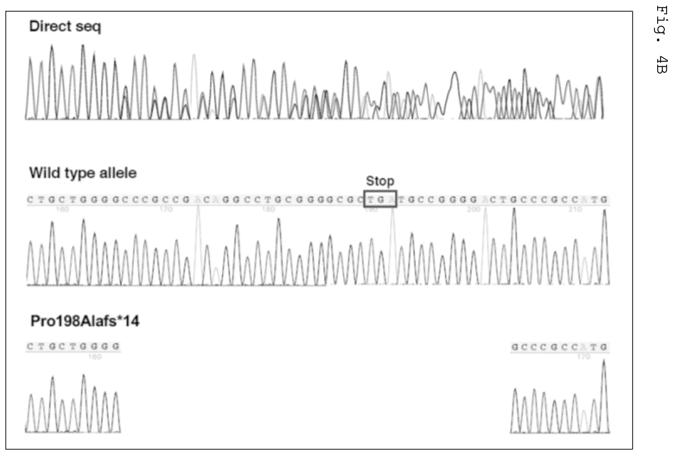

Fig. 4B is a diagram showing the genomic sequence and amino acid sequence of a mouse having an IL17D mutation. The top shows a result of direct sequencing for a mouse having an IL17D mutation. The middle shows a result of sequencing of a wild-type-derived allele isolated by cloning (SEQ ID NO: 11). The bottom shows a result of sequencing of a mutant-derived allele isolated by cloning (SEQ ID NO: 12). IL17D (NM_145837.3) c.592_615 del, p.Pro198Alafs*14.

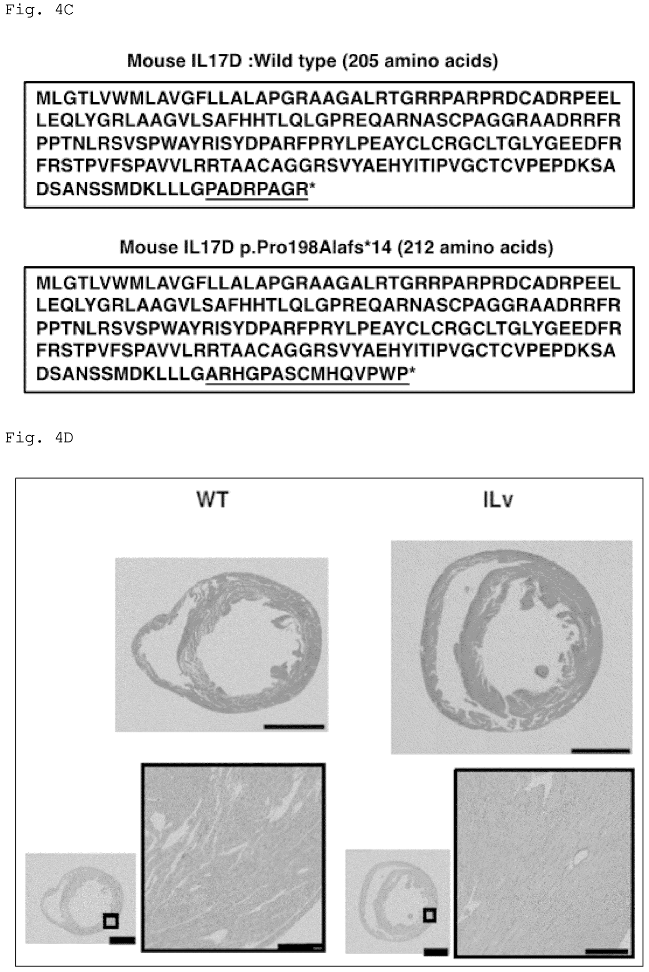

Fig. 4C is a diagram showing amino acid sequences of IL17D of a wild-type mouse (upper, SEQ ID NO: 7) and IL17D p.Pro198Alafs*14 (lower, SEQ ID NO: 13). While the wild-type IL-17D consists of 205 amino acids, IL17D p.Pro198Alafs*14 is predicted to have 212 amino acids.

Fig. 4D is a typical image showing results of observing cross-sections of the heart of a mouse having Pro198Alafs*14/+. In Fig. 4D, "WT", photographs on the left, show results of observing a cross-section of the heart of a 6-month-old wild-type mouse, and "ILv", photographs on the right, show results of observing a cross-section of the heart of an IL17D mutant mouse. The top shows results of observation with HE staining, and the bottom shows results of observation with Picro-sirius red staining and their magnified images. Scale bars in the magnified images represent 0.2 mm, and scale bars in other images represent 2 mm.

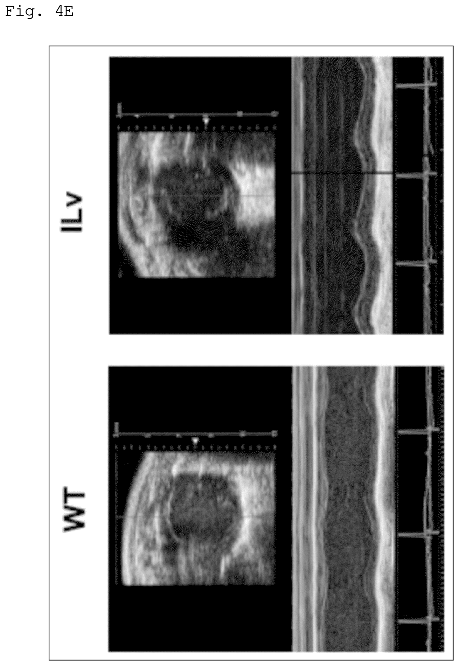

Fig. 4E represents images showing typical results of echocardiographic analysis of mice (11-week-old). In Fig. 4E, "WT", a photograph on the left, indicates a wild-type mouse, and "ILv", a photograph on the right, indicates an IL17D mutant mouse. The top shows A-mode images at end-diastole, and the bottom shows M-mode images.

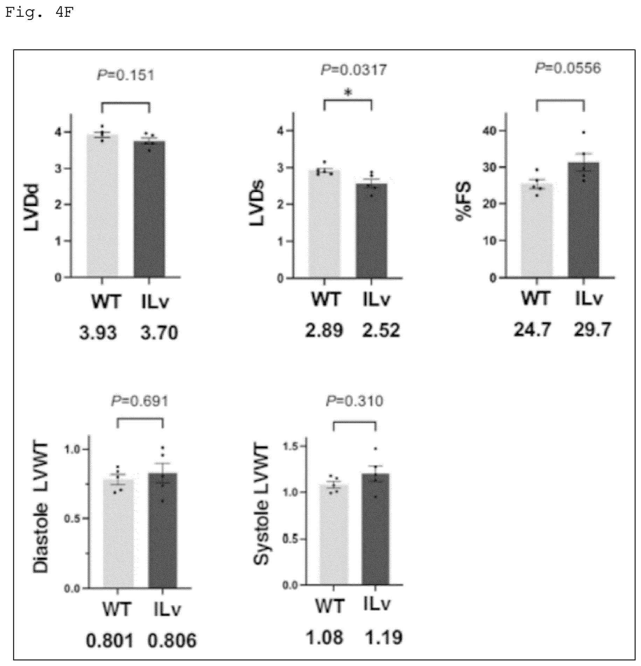

Fig. 4F represents graphs showing results of echocardiographic analysis of mice (11-week-old). LVDd: left ventricular end-diastolic diameter, LVDs: left ventricular end-systolic diameter, %FS: fractional shortening, LVWT: left ventricular wall thickness. In each graph, a numerical value under the letter "WT" or "ILv" represents an average value of each value.

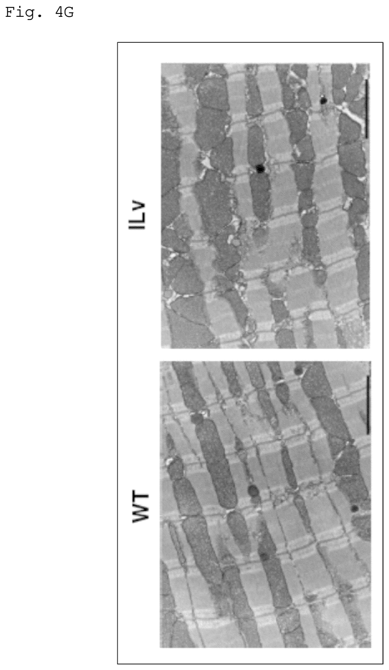

Fig. 4G represents typical images showing results of observing myocardia of mice (12-week-old) in electron-microscopic images. In Fig. 4G, "WT", a photograph on the left, indicates a wild-type mouse, and "ILv", a photograph on the right, indicates an IL17D mutant mouse.

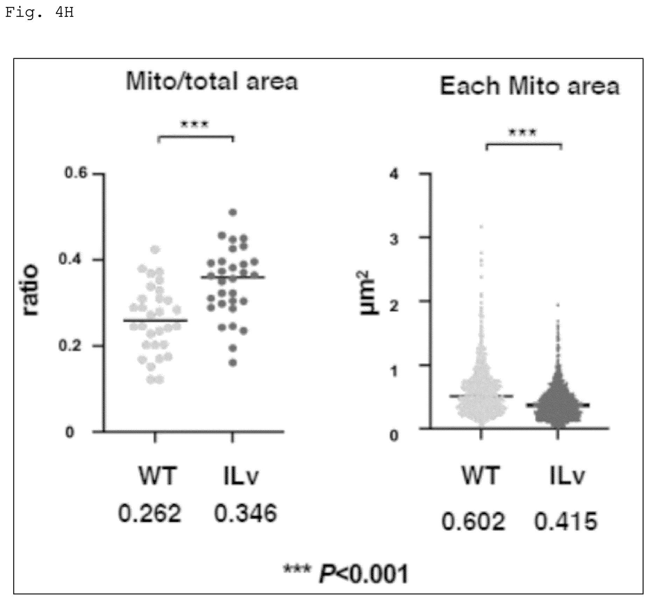

Fig. 4H represents graphs showing results of analyzing myocardia of mice (12-week-old) in electron-microscopic images. The left graph shows areas occupied by mitochondria in each image (ratios). The right graph shows areas of individual mitochondria.

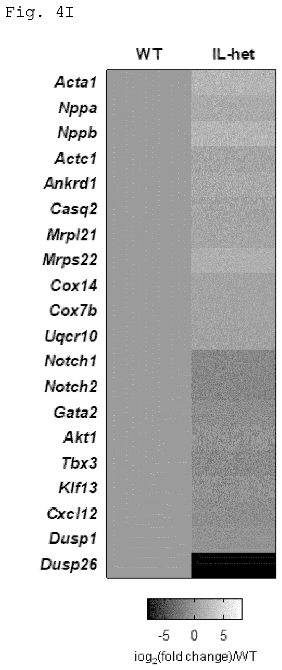

Fig. 4I is a heatmap showing typical genes exhibiting significant differences between a mouse having IL17D p.Pro198Alafs*14/+ myocardia and a mouse having wild-type myocardia in RNA-seq.

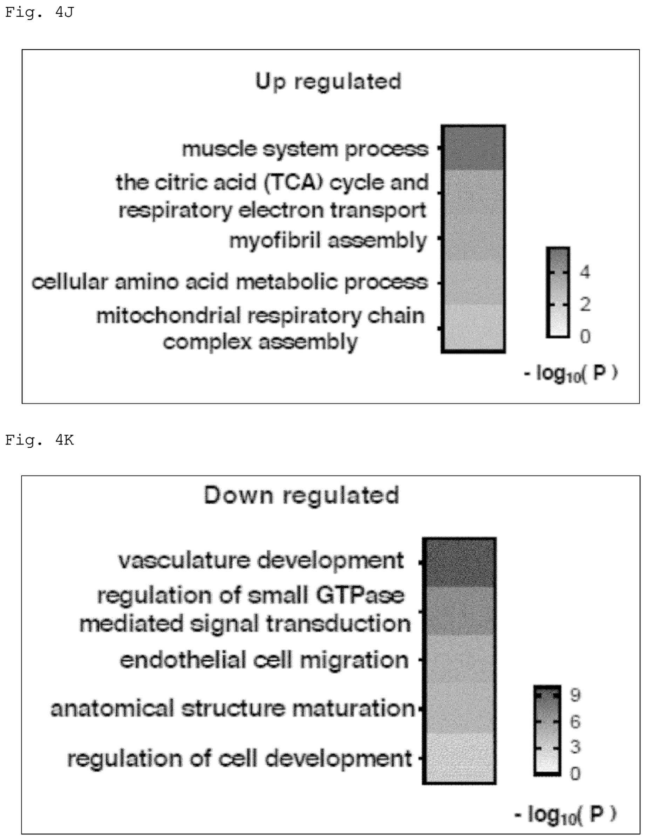

Fig. 4J is a heatmap showing results of Gene Ontology analysis for genes found to be upregulated in RNA-seq analysis.

Fig. 4K is a heatmap showing results of Gene Ontology analysis for genes found to be downregulated in RNA-seq analysis.

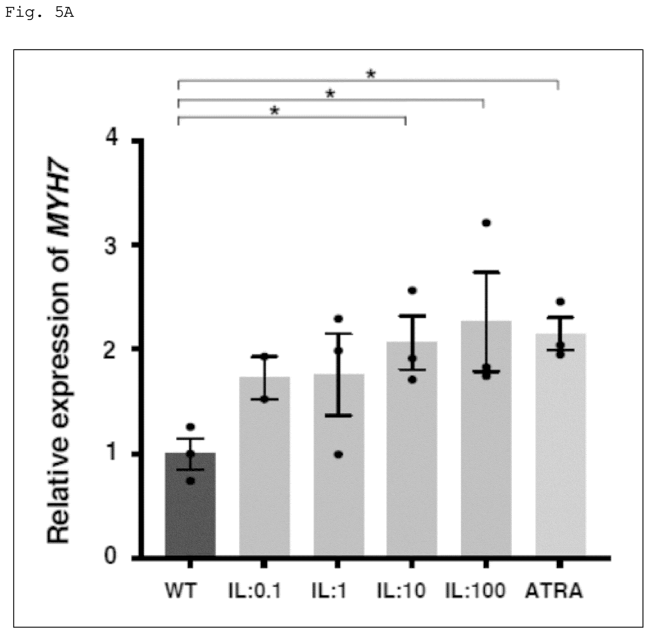

Fig. 5A is a graph showing transition of the relative expression level of MYH7 gene in H9c2 cells. Normal H9c2 cells are indicated as "WT". H9c2 cells treated with 0.1 ng/mL to 100 ng/mL IL-17D are indicated as "IL:0.1" to "IL:100", respectively. H9c2 cells differentiated with 100 nM ATRA are indicated as "ATRA".

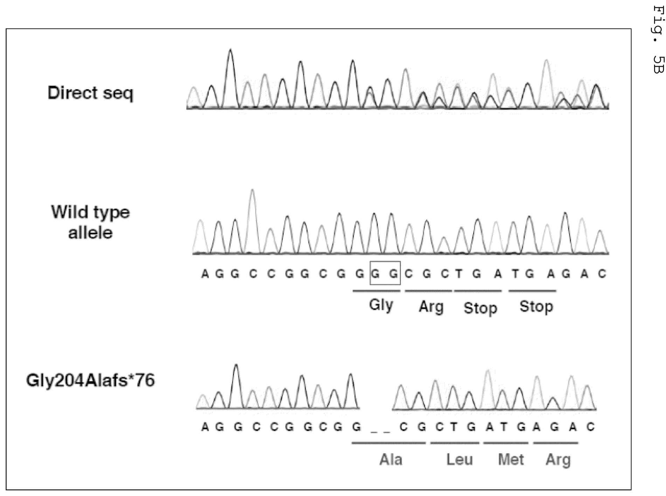

Fig. 5B is a diagram showing the genomic sequence and amino acid sequence of H9c2 cells having a read-through IL17D variant produced by genome editing. The top shows a result of direct sequencing for H9c2 cells subjected to the genome editing. The middle shows a result of sequencing of a wild-type-derived allele isolated by cloning (SEQ ID NO: 14). The bottom shows a result of sequencing of a mutant-derived allele isolated by cloning (SEQ ID NO: 15). Rat IL17D c.610_611del, p.Gly204Alafs*76.

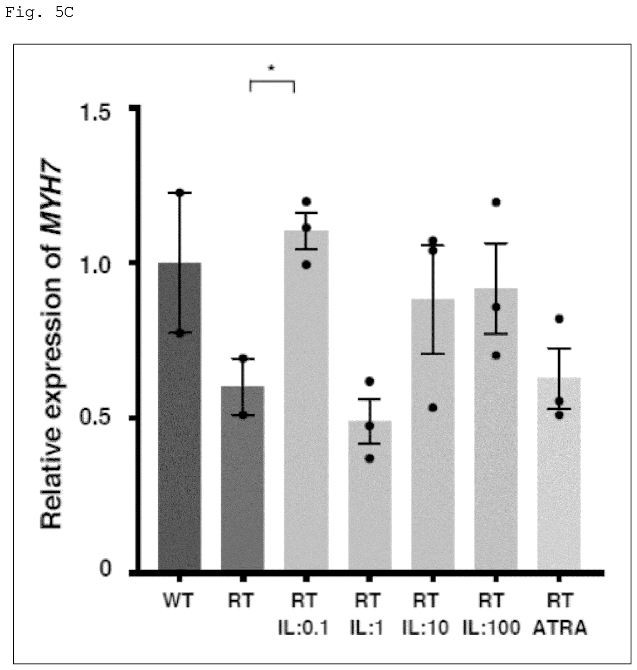

Fig. 5C is a graph showing transition of the relative expression level of MYH7 gene in H9c2 cells. Normal H9c2 cells are indicated as "WT". H9c2 cells having a read-through mutation of IL17D (IL17D p.Gly204Alafs*76/+) are indicated as "RT". The mutant H9c2 cells treated with 0.1 ng/mL to 100 ng/mL IL-17D are indicated as "RT IL:0.1" to "RT IL:100", respectively. H9c2 cells differentiated with 100 nM ATRA are indicated as "ATRA".

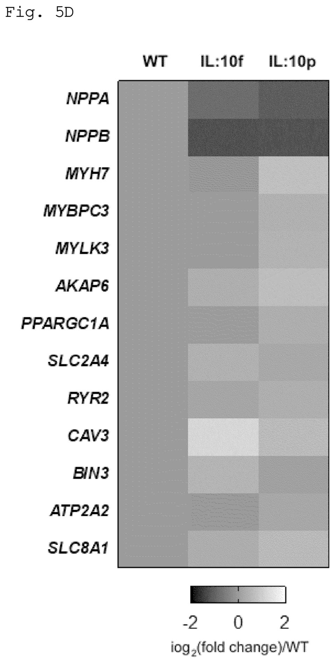

Fig. 5D is a heatmap showing results of RNA-seq analysis for hiPS-CMs as a control ("WT" in Fig. 5D) and hiPS-CMs treated with 10 fg/mL or 10 pg/mL human IL-17D ("IL:10f" or "IL:10p" in Fig. 5D).

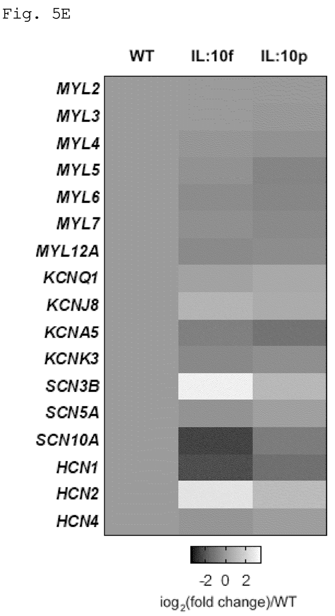

Fig. 5E is a heatmap showing results of RNA-seq analysis for hiPS-CMs as a control ("WT" in Fig. 5E) and hiPS-CMs treated with 10 fg/mL or 10 pg/mL human IL-17D ("IL:10f" or "IL:10p" in Fig. 5E).

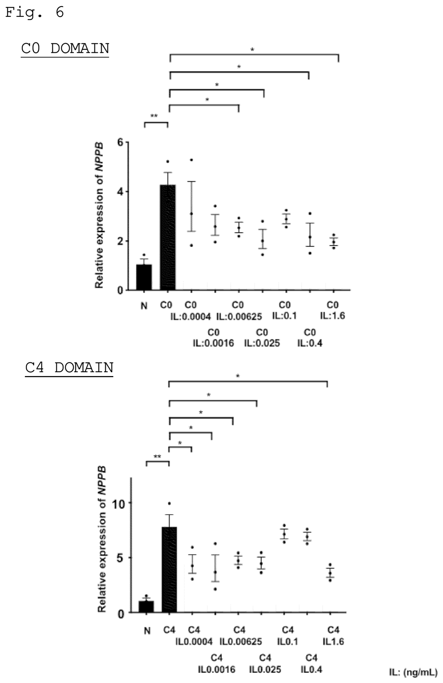

Fig. 6 represents graphs showing results of subjecting hiPS-CMs to genome editing for MYBPC3 gene, which is found most frequently as a cause of hypertrophic cardiomyopathy, administering IL-17D, and analyzing relative expression of NPPB gene. In Fig. 6, hiPS-CMs are indicated as "N". Cells subjected to genome editing for C0 domain of MYBPC3 are indicated as "C0". Cells subjected to genome editing for C4 domain of MYBPC3 are indicated as "C4". In addition, cells subjected to the genome editing and rescued with administration of 0.0004 ng/mL to 1.6 ng/mL IL-17D are indicated as "C0 IL:0.0004" to "C0 IL:1.6" and "C4 IL:0.0004" to "C4 IL:1.6", respectively.

Description of Embodiments

(Composition for suppressing Cardiac Hypertrophy)

The present invention provides a composition for suppressing cardiac hypertrophy, including interleukin-17D as an active ingredient.

In the present invention, the term "interleukin-17D" refers to a cytokine referred to as IL-17D, and the gene encoding the protein is also represented by IL17D. IL-17D is identified by UniProt ID: Q8TAD2 when derived from a human. IL-17D is also identified as a protein including the amino acid sequence identified as NCBI Reference Sequence: NP_001372152, NP_001372153, or NP_612141 (isoform c, a protein including the amino acid sequence encoded by the DNA sequence identified by NM_001385223, NM_001385224, or NM_138284), a protein including the amino acid sequence identified by NCBI Reference Sequence: NP_001372150 (isoform a, a protein including the amino acid sequence encoded by the DNA sequence identified by NM_001385221), a protein including the amino acid sequence identified by NCBI Reference Sequence: NP_001372151 (isoform b, a protein including the amino acid sequence encoded by the DNA sequence identified by NM_001385222), a protein including the amino acid sequence identified by NCBI Reference Sequence: NP_001372154 (isoform d, a protein including the amino acid sequence encoded by the DNA sequence identified by NM_001385225), or a protein including the amino acid sequence identified by NCBI Reference Sequence: NP_001373902 (isoform e, a protein including the amino acid sequence encoded by the DNA sequence identified by NM_001386973).

In the present invention, the term "cardiac hypertrophy" means at least that a cardiac hypertrophy marker has a high value. Examples of the cardiac hypertrophy marker include brain natriuretic peptide (BNP), atrial natriuretic peptide (ANP), skeletal muscle actin (actin $\alpha 1$, ACTA1), and mitochondrial genes (Mrp gene cluster, Cox gene cluster). Cardiac hypertrophy according to the present invention may also include a state of myocardia being thickened in the heart, more specifically, a pathological state of myocardia having an increased capacity beyond the range of physiological hypertrophy. A site having thickened myocardia is not particularly limited, and examples thereof include the left ventricle, the right ventricle, both ventricles, the interventricular septum, the right atrium, and the left atrium.

The meaning of the term "suppression of cardiac hypertrophy" encompasses not only partial suppression but also full suppression (inhibition) of cardiac hypertrophy. Examples thereof include reduction in level of a cardiac hypertrophy marker (the level of a cardiac hypertrophy marker reaching equal to or less than a reference value (normal value) or the like), and reduction in thickness of myocardia (the thickness of myocardia falling within a reference range (normal range) or the like).

The composition of the present invention may take a form of, for example, a pharmaceutical composition, or a reagent used for a research purpose (e.g., an *in vitro* or *in vivo* experiment).

The pharmaceutical composition of the present invention is more specifically a pharmaceutical composition for treating or preventing a disease associated with cardiac hypertrophy. In the present invention, the "disease associated with cardiac hypertrophy" is not particularly limited as long as the disease is a disease of the heart in which the above-mentioned cardiac hypertrophy is found, or a disease caused by the above-mentioned cardiac hypertrophy, and examples thereof include hypertrophic cardiomyopathy (HCM), dilated cardiomyopathy (DCM), secondary cardiomyopathy (e.g., amyloidosis or Fabry disease), hypertensive cardiac hypertrophy, valvular cardiac hypertrophy (e.g., valvular cardiac hypertrophy mainly related to the aortic valve).

In the present invention, a "subject" administered IL-17 is not particularly limited, and is an animal including a human. As an animal other than a human (non-human animal), for example, a domestic animal, a fowl, a pet, or a laboratory animal may be a subject. Specific examples thereof include pigs, bovines, horses, sheep, goats, chickens, wild ducks, ostriches, ducks, dogs, cats, rabbits, hamsters, mice, rats, and monkeys, but the subject according to the present invention is usually a human and is not limited in gender, age, race, or the like. More specific examples thereof include a human having a disease associated with cardiac hypertrophy, a human with a risk of having a disease associated with cardiac hypertrophy, a human with a risk of recurrence of a disease associated with cardiac hypertrophy, and a human with recurrence of a disease associated with cardiac hypertrophy.

In the present invention, the term "treatment" encompasses mitigating cardiac hypertrophy or an accompanying symptom thereof (alleviation of a symptom), blocking or delaying exacerbation of cardiac hypertrophy, improving an abnormal clinical laboratory value associated with cardiac hypertrophy (e.g., BNP described above), and the like. The term "prevention" also encompasses prevention, delay, or reduction in risk of recurrence of cardiac hypertrophy.

The "pharmaceutical composition" of the present invention may include any other pharmacologically acceptable component in addition to IL-17D. Examples of such other component include carriers, emulsifiers, humectants, pH buffering agents, media, excipients, disintegrants, buffering agents, tonicity modifiers, suspending agents, solubilizers, soothing agents, stabilizers, preservatives, and antiseptics. More specifically, in a liquid preparation such as an injectable, examples of the other pharmacologically acceptable component may include aqueous solutions (e.g., saline, water for injection, phosphate buffers, aqueous glucose solutions, and aqueous glycerol solutions) and aluminum hydroxide. In a lyophilized preparation, examples thereof may include, but are not limited to, sugars (e.g., mannitol, lactose, and saccharose) and albumin. Further, the pharmaceutical composition of the present invention may take a form of a kit including the above-mentioned components that may be mixed before administration. In addition, in use as an injectable, the pharmaceutical composition may take a form introduced in a syringe. In addition, the present invention may use IL-17D itself to suppress cardiac hypertrophy and/or diastolic dysfunction, or may use IL-17D as a composition further including any other pharmacologically acceptable component in addition to IL-17D as described above. Accordingly, the present invention provides a suppressant for cardiac hypertrophy and/or diastolic dysfunction, including IL-17D. Details and methods of use of IL-17D in an embodiment of a suppressant for cardiac hypertrophy and/or diastolic dysfunction are the same as those of the pharmaceutical composition.

The pharmaceutical composition of the present invention may include only IL-17D as an active ingredient, or may include IL-17D and at least one of other drugs that may be used in treatment of cardiac hypertrophy. The antibody or the like of the present invention may be administered together with any other drug that may be used in treatment of cardiac hypertrophy. When used for such purposes, the other drug that may be used in treatment of cardiac hypertrophy may be administered to a subject simultaneously with, separately from, or sequentially to the IL-17D of the present invention, or may be administered with varying each interval of administration. Examples of such "drug that may be used in treatment of cardiac hypertrophy" include antihypertensives (ACE inhibitors and angiotensin II receptor antagonists).

An administration method for IL-17D or the pharmaceutical composition varies depending on the form of the composition, the age, weight, gender, and physical condition of a subject, or the like, but the administration may be performed by any one of administration routes including parenteral administration (e.g., subcutaneous administration, intravenous administration, intraarterial administration, or local administration) and oral administration. A preferred administration method is parenteral administration, and subcutaneous administration (e.g., subcutaneous injection) is more preferred. Rather than systemic administration, local administration may also be performed. An example of the local administration may be direct injection into a target tissue (e.g., the heart).

The dose of the pharmaceutical composition may vary depending on the age, weight, gender, and physical condition of a subject, the degree of progression of a symptom, and a component in the pharmaceutical composition to be administered. In addition, a schedule of administration may be appropriately adjusted depending on those conditions, and may include a single dose or a plurality of sequential or periodic doses. In addition, administration may be followed by monitoring of the degree of cardiac hypertrophy and the like, and then determination of the timing of administration on the basis of the results thereof.

(Examination Method for Cardiac Hypertrophy)

The present invention provides an examination method for cardiac hypertrophy, including a step of detecting IL-17D for a sample isolated from a test subject.

The "test subject" to be subjected to the examination of the present invention is not particularly limited, and may be an animal including a human as in the above-mentioned subject, but is usually a human. There is also no limitation in gender, age, race, or the like, and examples thereof include a person with a risk of having cardiac hypertrophy, a person with suspected cardiac hypertrophy, a person with a risk of having a disease associated with cardiac hypertrophy, and a person with a suspected disease associated with cardiac hypertrophy.

The "sample" to be subjected to the examination of the present invention is not particularly limited as long as IL-17D can be detected, and examples thereof include blood samples and urinary samples. Such sample in the present invention may be one of not only those themselves (e.g., blood (whole blood) and urine) but also components thereof (e.g., protein and nucleic acid), samples derived therefrom (e.g., serum and plasma), and samples containing each thereof, and may also be subjected to preliminary treatment before detection of IL-17D. Such preliminary treatment is not particularly limited, but examples thereof include addition of a treatment liquid (e.g., a solution supplemented with a buffer, and further, a chelator, and a surfactant), centrifugation, filtration, stirring, fractionation of a protein or a nucleic acid, extraction of a protein or a nucleic acid, precipitation of a protein or a nucleic acid, degradation of a protein (segmentation into peptides), heating, freezing, and refrigeration. A plurality of those treatments may also be performed in combination.

In the present invention, the "detection of IL-17D" may be quantitative detection or qualitative detection.

The "quantitative detection" means detecting the amount of IL-17D in the sample. The "amount of IL-17D" to be detected may be not only the amount as a protein but also the amount of a transcript (mRNA or cDNA) of IL17D gene reflected thereto. The amount to be detected may also be not only an absolute amount but also a relative amount. An example of the relative amount is the ratio of the amount of a protein or the ratio of the amount of a transcript (a numerical value represented by a so-called arbitrary unit (AU)) based on a measuring method or a measuring device used in detection. As the relative amount, for example, a value calculated on the basis of the amount of another protein or transcript may also be used.

The "detection of the amount of a protein" may be performed by appropriately adopting a known technique by a person skilled in the art. Examples of such known technique include a detection method with an antibody (immunological method) and mass spectrometry. In the "immunological method", an antibody that recognizes IL-17D is used to bring the antibody into contact with IL-17D in a sample and detect the amount of the protein of IL-17D by using the binding property of the antibody to IL-17D as an indicator. Examples of the immunological method include immunochromatography, enzyme-linked immunosorbent assay (ELISA), CLEIA (chemiluminescence enzyme immunoassay), latex agglutination, multiplex assay, sandwich methods such as antibody array, immunoblotting, immunoaffinity chromatography, imaging cytometry, flow cytometry, radioimmunoassay, immunoprecipitation, immunohistochemical staining, and surface plasmon resonance (SPR).

The "detection of the amount of a transcript" may also be performed by appropriately adopting a known technique by a person skilled in the art. As such known technique, quantification may be performed by, for example, PCR (RT-PCR, real-time PCR, or quantitative PCR), DNA microarray analysis, Northern blotting, next-generation sequencing (sequencing-by-synthesis (e.g., sequencing with Solexa genome analyzer or Hiseq (trademark) 2000 manufactured by Illumina, Inc), Pyrosequencing (e.g., sequencing with the sequencer GSLX or FLX available from Roche Diagnostics K.K. (454) (so-called 454 sequencing)), ligase reaction sequencing (e.g., sequencing with SoliD (trademark) or 5500xl manufactured by Life Technologies Corporation), T-RFLP, bead array, *in situ* hybridization, dot blot, RNase protection assay, mass spectrometry, genomic PCR, or Southern blotting.

When the amount of IL-17D thus detected is consequently larger than a reference amount, the test subject can be determined to be in a state of cardiac hypertrophy, to have a risk of cardiac hypertrophy, or to have a disease associated with cardiac hypertrophy or a risk thereof.

The "reference amount" of IL-17D, which is to be a target for comparison, is a so-called cutoff value, and examples thereof include a value determined by comparing the amount of IL-17D between a human population having no disease associated with cardiac hypertrophy and a human population having a disease associated with cardiac hypertrophy (e.g., a value set between the amount of IL-17D in a human population having no disease associated with cardiac hypertrophy (the median, average value, or upper limit value) and that in a human population having a disease associated with cardiac hypertrophy (the median, average value, or lower limit value)). In addition, the setting may be performed by a person skilled in the art by appropriately selecting a statistical analysis method. Examples of the statistical analysis method include receiver operating characteristics analysis (ROC analysis), t-test, analysis of variance (ANOVA), Kruskal-Wallis test, Wilcoxon test, Mann-Whitney test, odds ratio, hazard ratio, Fisher's exact test, and classification tree and decision tree analysis (CART analysis). Normalized or standardized and normalized data may also be used for the comparison.

The "qualitative detection" means, for example, detection of a sequence of IL17D, and a more specific example thereof is analyzing a nucleotide sequence of IL17D gene or an expression control region thereof and thereby detecting a mutation that leads to suppression of a function of IL-17D.

The "analysis of a nucleotide sequence" and the "detection of a mutation" may be performed by a person skilled in the art by appropriately adopting a known technique. Examples of such known technique include Maxam-Gilbert method, Sanger method, and next-generation sequencing described above. Examples thereof also include CAPS (PCR-RFLP), high-resolution melt curve analysis (HRM), double-dye probing (e.g., TaqMan (trademark) probing), PCR-sequence specific primer (PCR-SSP) method, and PCR-single stranded conformation polymorphism (PCR-SSCP).

The "suppression of a function of IL-17D" means suppression of the cardiac hypertrophy-suppressing function and/or cardiomyocyte differentiation potential of IL-17D. The suppression of a function also encompasses not only partial suppression but also full suppression (inhibition). Such suppression of a function may be evaluated by a person skilled in the art by, for example, a method shown in Examples to be described later. The suppression of a function also encompasses suppression of expression of IL-17D, which contributes to the function.

The "type of a mutation" that provides suppression of the above-mentioned function is not particularly limited. Examples thereof include substitution, deletion, addition, and/or insertion of a nucleotide, and may also include frameshift mutation, nonsense mutation, null mutation, in-frame mutation, inversion, and translocation. "The number of mutations" in IL17D gene or an expression control region thereof is also not particularly limited as long as the above-mentioned function can be suppressed, and may be one or a plurality of mutations (e.g., 2, 3 or less, 5 or less, 10 or less, 20 or less, 30 or less, 40 or less, and 50 or less).

A "site" to which such mutation is introduced is not particularly limited as long as the above-mentioned function can be suppressed, but examples thereof include a nucleotide sequence encoding an amino acid that is highly conservative in a sequence of IL-17D. Examples of the "amino acid that is highly conservative" herein include an amino acid in a site identical among at least 5 species (preferably 6 species, more preferably 7 species, still more preferably 8 species, particularly preferably 9 species) of animals in the alignment of amino acid sequences of IL-17D derived from 9 species of animals shown in Fig. 1E.

More specific examples of the mutation according to the present invention include substitution of tyrosine at position 43 with another amino acid (preferably substitution for histidine), substitution of glutamic acid at position 64 with another amino acid (preferably substitution for valine), substitution of proline at position 72 with another amino acid (preferably substitution for leucine), substitution of lysine at position 175 with another amino acid (preferably substitution for arginine) in an amino acid sequence of human-derived IL-17D as shown in Examples to be described later. The "substitution with another amino acid" herein refers to substitution with an amino acid different from that of wild-type, and is not particularly limited as long as the substitution leads to suppression of a function of IL-17D, and a preferred example thereof is nonconservative substitution.

As shown in Examples to be described later, an example thereof may also be addition of an amino acid at the C terminal of IL-17D. The number of amino acids added is not particularly limited as long as the addition of the amino acids leads to suppression of a function of IL-17D, but examples thereof include 5 amino acids or more, 10 amino acids or more, 20 amino acids or more, and 30 amino acids or more.

When a mutation that suppresses a function of IL-17D is consequently detected, the test subject can be determined to be in a state of cardiac hypertrophy, to have a risk of cardiac hypertrophy, or to have a disease or a potential disease associated with cardiac hypertrophy.

Such examination is usually performed by a physician (including a person directed by a physician), and the data related to IL-17D as described above is helpful for diagnosis by a physician including judgment of the necessity for treatment and the timing thereof and the like. The method of the present invention may be represented as a method of collecting data of IL-17D for examination for cardiac hypertrophy by a physician, a method of presenting the data to a physician, a method of performing analysis by comparing IL-17D and a reference amount or a sequence, and a method for aiding diagnosis of cardiac hypertrophy by a physician.

(Drug for examining Cardiac Hypertrophy)

As described above, in the present invention, cardiac hypertrophy may be examined by detecting IL-17D as a gene (sequence), a transcript, or a protein. Accordingly, the present invention is directed to a drug for examining cardiac hypertrophy by the above-mentioned examination method, and provides a drug (testing drug) containing at least one compound selected from the following items (a) and (b): (a) an oligonucleotide that has at least 15 nucleotides in chain length and hybridizes to IL17D gene, a transcript thereof, or a complementary nucleic acid thereof, and (b) an antibody that binds to IL-17D protein.

An oligonucleotide in the drug of the present invention may have a form of a primer or a form of a probe in accordance with the above-mentioned detection method.

A primer in the drug of the present invention is not particularly limited as long as the primer hybridizes to IL17D gene, a transcript thereof, or a complementary nucleic acid thereof and enables amplification or detection thereof. The primer may be formed of only DNAs, or may have partial or full substitution with an artificial nucleic acid(s) (a modified nucleic acid(s)) such as a bridged nucleic acid(s). The size of the primer needs to be at least about 15 nucleotides or more in length, and is preferably 15 to 100 nucleotides in length, more preferably 18 to 50 nucleotides in length, still more preferably 20 to 40 nucleotides in length. The number of primers required varies depending on the type of the above-mentioned detection method, and the number of primers in the drug of the present invention is thus not particularly limited, but the drug of the present invention may contain two or more primers. Such primer may be designed and made by a person skilled in the art by a known method in accordance with the above-mentioned detection method.

The probe in the drug of the present invention is not particularly limited as long as the probe hybridizes to IL17D gene, a transcript thereof, or a complementary nucleic acid thereof and enables detection thereof. The probe may be DNAs, RNAs, artificial nucleic acids, or a chimeric molecule thereof. The probe may be any one of single-stranded and double-stranded probes. The size of the probe only needs to be at least about 15 nucleotides or more in length, preferably 15 to 1,000 nucleotides in length, more preferably 20 to 500 nucleotides in length, still more preferably 30 to 300 nucleotides in length. Such probe may be made by a person skilled in the art by a known method. The probe may be provided in a form fixed on a substrate such as microarray.

The antibody in the drug of the present invention is not particularly limited as long as the antibody can bind specifically to IL-17D protein. For example, an antibody against IL-17D protein may be any one of a polyclonal antibody and a monoclonal antibody, and may also be a functional fragment of an antibody (e.g., Fab, Fab', or scFv). Such antibody may be made by a person skilled in the art by a known method. The antibody may be provided in a form fixed on a substrate such as a plate so as to be used for ELISA, antibody array, or the like.

In addition, the oligonucleotide or the antibody in the drug of the present invention may be labeled with a labeling substance in accordance with the above-mentioned detection method. Examples of the labeling substance include: fluorescent substances, such as FITC, FAM, DEAC, R6G, TexRed, and Cy5; enzymes, such as β-D-glucosidase, luciferase, and HRP; radioisotopes, such as $^3$H, $^{14}$C, $^{32}$P, $^{35}$S, and $^{123}$I; affinity substances, such as biotin and streptavidin; and luminescent substances, such as luminol, luciferin, and lucigenin.

The drug of the present invention may also contain any other component acceptable as a composition in addition to the above-mentioned oligonucleotide or antibody. Examples of such other component include carriers, excipients, disintegrants, buffering agents, emulsifiers, suspending agents, stabilizers, preservatives, antiseptics, saline, and secondary antibodies.

In addition, in addition to the above-mentioned drug of the present invention, a substrate required for detection of a label, a positive control or a negative control, a buffer used for dilution or washing of a sample, or the like may be combined, and may also form a kit for examining cardiac hypertrophy. Such kit may further include an instruction insert of the kit.

(Screening Method for Substance having Activity to suppress Cardiac Hypertrophy 1)

The present invention provides a screening method for a substance having activity to suppress cardiac hypertrophy, including the steps of: bringing cardiomyocytes having a suppressed function of IL-17D into contact with a test substance; detecting expression of a cardiac hypertrophy marker in the cardiomyocytes; and determining the test substance to have activity to suppress cardiac hypertrophy when the expression of the cardiac hypertrophy marker detected in the detecting step is reduced as compared to no contact with the test substance.

The "cardiomyocyte" according to the present invention is described later. The "suppression of a function of IL-17D", which is as described above, may be performed by introducing the above-mentioned mutation into IL17D gene, an expression control region thereof, or the like in cardiomyocytes. Introduction of the mutation may be achieved by a person skilled in the art by a known method. Examples of such known method include, but are not limited to: genome editing; knock out; a method of targeting a transcript with use of siRNA, antisense RNA, or RNA having ribozyme activity; physical mutagenesis; a method with a chemical mutagenesis agent; and a method of introducing transposon or the like into genomic DNA. Genome editing is a method of modifying a target gene by utilizing site specific nuclease (e.g., DNA double-strand break enzyme such as zinc-finger nuclease (ZFN), transcription-activator-like effector nuclease (TALEN), and CRISPR-Cas9).

In the screening method of the present invention, the "test substance" to be brought into contact with the cardiomyocytes having a suppressed function of IL-17D is not particularly limited as long as the test substance may contain a substance having activity to suppress cardiac hypertrophy, and examples thereof include: synthesized low-molecule compound libraries; expression products of gene libraries; peptide libraries; polynucleotide libraries; antibodies; bacterial released substances; extracts and culture supernatants of cells (microbes, plant cells, animal cells); purified or partially purified polypeptides; and extracts obtained from marine organisms, plants, or animals.

The "contact" of cardiomyocytes having a suppressed function of interleukin-17D with a test substance is not particularly limited, but may be usually performed by adding a test substance to a medium used for culturing the cardiomyocytes. The "medium" herein may also be prepared by a person skilled in the art on the basis of a known basal medium for culturing cardiomyocytes as described later.

Detection of expression of a cardiac hypertrophy marker in cardiomyocytes with or without contact with a test substance may be appropriately performed by a person skilled in the art by using a known method of detecting the amount of a transcript or a known method of detecting the amount of a protein in the same manner as in IL-17D described above. The "cardiac hypertrophy marker" to be detected is as described above. When expression of a cardiac hypertrophy marker detected in cardiomyocytes that experienced contact with a test substance is consequently reduced as compared to those having experienced no contact with the test substance, the test substance is determined to have activity to suppress cardiac hypertrophy.

(Screening Method for Substance having Activity to suppress Cardiac Hypertrophy 2)

The present invention provides a method including the steps of: feeding a test substance to a non-human animal having a suppressed function of interleukin-17D; detecting cardiac hypertrophy in the non-human animal; and determining the test substance to have activity to suppress cardiac hypertrophy when the cardiac hypertrophy detected in the detecting step is reduced as compared to no administration of the test substance.

The "suppression of a function of interleukin-17D" and the "non-human animal" are as described above. A non-human animal having the function suppressed may be prepared by a person skilled in the art by introducing the above-mentioned mutation into a non-human animal-derived germ cell by using a known method such as genome editing.

The "test substance" administered to a non-human animal having a suppressed function of interleukin-17D is as described above. The "administration" of the substance may be performed by a person skilled in the art by appropriately selecting oral administration, parenteral administration, or the like in accordance with the type, form, and the like of the test substance.

The "cardiac hypertrophy" detected in a non-human animal with or without administration of a test substance is as described above, and may be represented by, for example, the amount of a cardiac hypertrophy marker or the thickness of myocardia. When cardiac hypertrophy detected in a non-human animal administered a test substance is reduced as compared to that without administration of the test substance, the test substance is determined to have activity to suppress cardiac hypertrophy.

Embodiments according to cardiac hypertrophy in the present invention have been described so far, but the subject of the present invention is not limited to cardiac hypertrophy. As shown in Examples to be described later, a causal connection between IL-17D and hypertrophic cardiomyopathy (HCM) is revealed, and further, the use of IL-17D also enables treatment and the like of HCM. Herein, HCM is a primary myocardial disease characterized by hypertrophy and diastolic dysfunction of the left ventricle. Accordingly, IL-17D can deal with not only cardiac hypertrophy but also diastolic dysfunction.

That is, the present invention can also target diastolic dysfunction instead of or in addition to cardiac hypertrophy. Specifically, the present invention can provide the following embodiments.

[1] A composition for suppressing cardiac hypertrophy and/or diastolic dysfunction, including interleukin-17D as an active ingredient.

[2] The pharmaceutical composition according to Item [1], wherein the pharmaceutical composition is a pharmaceutical composition for treating or preventing a disease associated with cardiac hypertrophy and/or diastolic dysfunction.

[3] An examination method for cardiac hypertrophy and/or diastolic dysfunction, including a step of detecting interleukin-17D for a sample isolated from a test subject.

[4] A screening method for a substance having activity to suppress cardiac hypertrophy and/or diastolic dysfunction, including the steps of: bringing cardiomyocytes having a suppressed function of interleukin-17D into contact with a test substance; detecting expression of a cardiac hypertrophy marker and/or a diastolic dysfunction marker in the cardiomyocytes; and determining the test substance to have activity to suppress cardiac hypertrophy and/or diastolic dysfunction when the expression of the cardiac hypertrophy marker and/or the diastolic dysfunction marker detected in the detecting step is reduced as compared to no contact with the test substance.

[5] A screening method for a substance having activity to suppress cardiac hypertrophy and/or diastolic dysfunction, including the steps of: feeding a test substance to a non-human animal having a suppressed function of interleukin-17D; detecting cardiac hypertrophy and/or diastolic dysfunction in the non-human animal; and determining the test substance to have activity to suppress cardiac hypertrophy and/or diastolic dysfunction when the cardiac hypertrophy and/or the diastolic dysfunction detected in the detecting step is reduced as compared to no feeding of the test substance.

The "diastolic dysfunction" according to the present invention means a state in which a function of myocardia is impaired by a decrease in diastolic ability and compliance of myocardia mainly associated with the ventricle.

Examples of the "diastolic dysfunction marker" according to the present invention include a decrease in sarcomere length in cardiomyocytes at end-diastole, an increase in intra-cardiomyocyte Ca concentration at end-diastole, an increase in left ventricular end-diastolic pressure, and enlargement of left atrial diameter.

Examples of the "disease associated with diastolic dysfunction" according to the present invention include: hypertrophic cardiomyopathy; restrictive cardiomyopathy; dilated cardiomyopathy; primary cardiomyopathy such as left ventricular noncompaction; secondary cardiomyopathy such as amyloidosis or Fabry disease; ischemic myocardial disorder; metabolic myocardial disease associated with diabetes or thyroid dysfunction; hypertensive cardiac hypertrophy; myocardial disorder caused by a drug having cardiotoxicity such as adriamycin; inflammatory myocardial disease caused by virus or immunological mechanism; and valvulopathic myocardial disease represented by aortic valve stenosis.

(Production Method for Differentiated Cardiomyocytes)

The present invention also provides a production method for differentiated cardiomyocytes, including culturing immature cardiomyocytes in the presence of interleukin-17D.

The term "cardiomyocyte" in the present invention may encompass myocytes forming muscle of the heart (myocardia), and cell lineages thereof, and particularly both immature cardiomyocytes and differentiated cardiomyocytes. Cardiomyocytes may be divided into subtypes, and examples thereof include ventricle cardiomyocytes, atrium cardiomyocytes, and sinoatrial nodal cardiomyocytes.

The term "Immature" in the present invention refers to a state relative to differentiation, and means the degree of differentiation as cardiomyocytes is immature. Examples thereof include a state of low expression of a myocardia specific marker. Examples of the "myocardia specific marker" herein include GLUT4, SERCA2, CAV3, RyR2, and sodium/calcium exchangers. Examples of the "immature cardiomyocyte" include immature cardiomyocytes through differentiation induction from pluripotent stem cells, or cardiomyoblasts.

The term "pluripotent stem cell" means a cell having multipotency, which enables differentiation to any cell constituting an adult body, and self-renewal potential, which allows maintenance of the multipotency even through cell division. A pluripotent stem cell may be newly or previously established. A pluripotent stem cell encompasses embryonic pluripotent stem cells (ES cells), embryonic germ cells (EG cells), and artificial pluripotent stem cells (iPS cells).

Differentiation induction from a pluripotent stem cell to a cardiomyocyte is not particularly limited, and may be performed by a person skilled in the art by appropriately using a known technique. Examples thereof include: a method of culturing pluripotent stem cells in a medium for differentiation induction to myocardia, such as RPMI/B27 or IMDM; a method of culturing pluripotent stem cells in a medium containing a GSK3 inhibitor or a WNT signal activator and no insulin (sometimes containing a ROCK inhibitor), then culturing them in a medium containing a WNT signal inhibitor and no insulin, and further culturing them in a medium containing insulin; a method of culturing pluripotent stem cells in a medium containing a GSK3 inhibitor or a WNT signal activator and a PKC activator, and then culturing them in a medium containing a WNT signal inhibitor, a Src inhibitor, and an EGF receptor inhibitor (e.g., methods described in WO 2013/111875 A1, WO 2015/182765 A1, Minami I, et al., Cell Rep. 2012 Nov 29; 2(5): 1448-60, and Lian, X. et al., Nat. Protoc. 8, 162-175 (2013)).

The term "cardiomyoblast" means a cell that has self-renewal potential and is destined to differentiate to a cardiomyocyte, and examples thereof include rat heart-derived H9c2 cells.

In the present invention, such immature cardiomyocytes can be cultured in the presence of IL-17D, thereby promoting differentiation of the cardiomyocytes as shown in Examples to be described later. Culturing in the presence of IL-17D is usually performed by using a medium supplemented with IL-17D. Such "medium" supplemented with IL-17D is not particularly limited as long as cardiomyocytes can be maintained, and the medium may be prepared on the basis of a known basal medium for culturing cardiomyocytes. Examples of the known basal medium include Dulbecco's modified Eagle medium (DMEM), Williams E medium, Ham's F12 medium, KSOM medium, Eagle's MEM medium, Glasgow's MEM medium, αMEM medium, Ham's medium, RPMI 1640 medium, Fischer's medium, BME medium, BGJb medium, CMRL 1066 medium, MEM Zinc Option modified medium, IMDM medium, Medium 199 medium, and any mixture medium thereof. A medium according to the present invention may be a serum-containing medium or a serum-free medium. A serum-free medium means a medium containing no unadjusted or unpurified serum, and examples thereof include a medium containing a purified blood-derived component, an animal tissue-derived component (e.g., growth factor), or the like. A medium according to the present invention may also contain a serum replacement. Examples of the serum replacement include commercially available products, such as KnockOut™ Serum Replacement (KSR, manufactured by Invitrogen), Chemically-defined Lipid concentrated (manufactured by Gibco), and GlutaMAX™ (manufactured by Gibco). A medium according to the present invention may also contain an amino acid (e.g., L-glutamine or a non-essential amino acid), hormone (e.g., progesterone or β-estradiol), a growth factor (e.g., insulin, transferrin, selenite, or ITS), a reductant (e.g., β-mercaptoethanol), an antibiotic (e.g., penicillin or streptomycin), organic acid (e.g., pyruvic acid, sodium pyruvate, or lactic acid), fatty acid (e.g., linoleic acid) or lipid, sugars, vitamin, cytokine, an antioxidant, a buffer (e.g., HEPES), inorganic salts, and the like.

The concentration of IL-17D added to such medium is not particularly limited as long as differentiation of cardiomyocytes can be promoted, and the concentration is, for example, from $10^{-15}$ g/mL to $10^{-6}$ g/mL. More specifically, when iPS cell-derived cardiomyocytes are set as a subject, the concentration of IL-17D added to a medium is, for example, from $10^{-12}$ g/mL to $10^{-10}$ g/mL (preferably $10^{-11}$ g/mL (10 pg/mL)) or from $10^{-15}$ g/mL to $10^{-13}$ g/mL (preferably $10^{-14}$ g/mL (10 fg/mL)) as shown in Examples to be described later. When H9c2 cells are set as a subject, the concentration of IL-17D added to a medium is, for example, from $10^{-11}$ g/mL to $10^{-6}$ g/mL (preferably from $10^{-10}$ g/mL to $10^{-7}$ g/mL (from 0.1 ng/mL to 100 ng/mL)) as shown in Examples to be described later.

Further, another condition for promoting differentiation of cardiomyocytes may be appropriately selected and adjusted by a person skilled in the art based on known culture conditions for cardiomyocytes. For example, cultured temperature is not particularly limited, but is usually from about 30°C to about 40°C, preferably about 37°C. A $CO_2$ concentration is usually from about 1% to about 10%, preferably from about 2% to about 5%. An oxygen concentration is usually from 1% to 10%. In addition, culture of cardiomyocytes may be performed on extracellular matrix constituent protein. Examples of the extracellular matrix constituent protein include fibronectin, laminin, collagen, elastin, and a mixture thereof.

Differentiated cardiomyocytes produced by such culture are not limited, but in the present invention, particular examples thereof include ventricular cardiomyocytes.

Examples

The present invention is more specifically described below on the basis of Examples, but the present invention is not limited to the following Examples. Examples were performed by using materials and methods shown below.

(Human Sample)

All hypertrophic cardiomyopathy (HCM) patients and families thereof set as subjects in Examples lived in Japan. Patients having a complication, such as a neuromuscular disease or a teratologic syndrome, were excluded from the subjects. For gene analysis, documented informed consent was obtained from the patients and the families thereof. This human genetic study was approved by the ethical review board of Medical Research Institute, Tokyo Medical and Dental University.

(Whole Exome Sequencing and Evaluation of Each Variant in HCM Family)

Genomic DNA was purified from a human blood sample with Wizard DNA purification kit (Promega Corporation, WI, USA) in accordance with a manufacturer's manual (see Reference 10). The genomic DNA was analyzed by whole exome sequencing with Ion Torrent PGM™ system (Thermo Fisher Scientific, CA, USA). A DNA library was prepared with TargetSeq™ Exome Enrichment Kit (Thermo Fisher Scientific, Waltham, MA, USA) in accordance with a manual.

In brief, 3 μg of the genomic DNA was fragmented to 200 bp with Covaris ultrasonicator (Covaris Inc., Woburn, MA, USA), and purified with Ampure XP beads (Beckman Coulter, Brea, CA, USA). The blunt-end was ligated with an adaptor, and size selection was performed with E-Gel agarose gel. The DNA library thus selected was amplified and purified. The library was quantitatively and qualitatively analyzed with Agilent Bioanalyzer (Agilent Technologies, Santa Clara, CA, USA), and 500 ng of the library was hybridized with an Ion TargetSeq™ probe pool. The library hybridized with the probe was captured, amplified, and purified. The captured library was analyzed with Agilent Bioanalyzer. The purified library was subjected to emulsion PCR by an Ion One Touch 2 system with Ion PGM™ template OT2 200 kit (Thermo Fisher Scientific, Waltham, MA, USA). Ion Sphere Particles (ISPs) were concentrated with Ion One Touch ES (Thermo Fisher Scientific, Waltham, MA, USA). The prepared Ion Sphere Particles (ISPs) were loaded into an Ion 318 sequencing chip and subjected to sequencing with Ion PGM 200 Sequence Kit (Thermo Fisher Scientific, Waltham, MA, USA). Sequence data was analyzed with Torrent Suite 3.6 (Thermo Fisher Scientific, Waltham, MA, USA). Filtered reads were mapped to hg19, a reference genome, to extract variants. Each library was subjected to iterative sequencing, and variant analysis was performed at the time point when the average depth of combined sequence data exceeded 30.

Genomic DNA prepared from each of HCM patients ((II-1) and (II-2)), and a human having no onset of HCM even at the age of 40 or more (III-4) was analyzed by whole exome sequencing (Fig. 1A). For the extracted variants different from the reference genome, a variant carried by both the HCM patients (II-1) and (II-2) and not carried by the human having no HCM (III-4) was selected with a filter. A nonsynonymous variant was selected with CLC genomic workbench ver. 6 (Qiagen, Venlo, Netherlands). Further, a variant having a minor allele frequency (MAF) of less than 0.2% was extracted from 1000 Genomes Project (TG) and Human Genetic Variation Database (HGVD), which was used as a control variant database of a Japanese population (see References 7 and 9). All the selected variants were recognized by using Sanger sequencing. Finally, a variant commonly carried by all the HCM patients was selected.

(Gene Analysis of IL17D for HCM Probands by Sanger Sequencing)

Sanger sequencing was performed in order to identify a rare IL17D variant in 573 genetically unrelated HCM proband patients who had no cardiomyopathy-related mutation in eight genes (MYH7, MYBPC3, TNNT2, TNNI3, MYL3, MYL2, TPM1, and ACTC1) encoding sarcomere. PCR primer pairs were designed for all exons of IL17D as shown below: Exon1-F: TCCCATCCTCCGGCCGCCT (SEQ ID NO: 16), Exon1-RN: GCCAACCACCTCCTTCCCCG (SEQ ID NO: 17), Exon2-F: TCCCCGTGACTCTAACCAG (SEQ ID NO: 18), and Exon2-R: CACTCTTCAGGAGTCGCC (SEQ ID NO: 19).

Exon1-F and Exon1-RN for exon 1, and Exon2-F and Exon2-R for exon 2 were used to identify a variant of IL17D. A PCR product was purified with ExoSAP-IT (Affymetrix, Santa Clara, CA, USA), and subjected to sequencing with BigDye terminator v3.1 Cycle Sequencing Kit (Thermo Fisher Scientific, Waltham, MA, USA) on ABI 3130 (Thermo Fisher Scientific, Waltham, MA, USA). A mutation having a MAF of less than 0.002 (0.2%) was excluded in each database of TG, Exome aggregation consortium (ExAC), and HGVD (2-4).

Genomic DNA of a HCM patient having a rare IL17D variant was analyzed for 67 disease-related genes previously reported to have a relation to hereditary or secondary cardiomyopathy (ABCC9, ACTC1, ACTN2, ANKRD1, BAG3, CALR3, CAV3, CRYAB, CSRP3, DES, DOLK, DSG2, DSP, EMD, DTNA, EYA4, FHL1, FHL2, FHOD3, FKTN, DSP, DTNA, EMD, EYA4, FHL1, FHL2, FHOD3, FKTN, GAA, GATAD1, Gla, ILK, ISL1, JHP2, JUP, LAMA4, LAMP2, Ldb3, LMNA, MTO1, MURC, MIBPC3, MYH6, MYH7, MYL2, MYL3, MYLC2, MYOZ2, GPN, NEBL, EXN.OBSCN, NIKON, NYN, NIKON, NIKON, NIKON, NIKON, NIKN, OBSCN, PKP2, PLN, PRDM16, PRKAG2, PSEN1, PSEN2, RBM20, SCN5A, SDHA, SGCD, TAZ, TCAP, TGFB3, TIEG1, TMEM43, TMPO, TNNC1, TNNI3, TNNT2, TPM1, TTN, TTR, and VCL) with Ion Torrent PGM system (Thermo Fisher Scientific, Waltham, MA, USA) as reported in Reference 10.

In brief, 2,250 primer pairs were designed for the whole coding region of a target gene with AmpliSeq Designer (Thermo Fisher Scientific, Waltham, MA, USA). A DNA library was prepared with Ion Ampliseq Library Kit 2.0 (Thermo Fisher Scientific, Waltham, MA, USA). A total of 10 ng of genomic DNA was quantified with Qubit 2.0 Fluorometer and Qubit dsDNA assay kit (Thermo Fisher Scientific, Waltham, MA, USA), and amplified by multiplex PCR with the designed primers. The Ion Sphere Particles (ISPs) thus prepared were loaded into an Ion 318 sequencing chip, and subjected to base sequence determination with Ion PGM 400 Sequence Kit (Thermo Fisher Scientific, Waltham, MA, USA). The sequence data was analyzed with Torrent Suite 4.4 (Thermo Fisher Scientific, Waltham, MA, USA). Filtered reads were mapped to hg19, a reference genome, to extract variants. Further, a variant having a MAF of less than 0.2% in each of TG, ExAC, and HGVD was excluded with CLC genomic workbench ver. 6 (Qiagen, Venlo, Netherlands) (see References 7, 9, and 34). Functional changes related to a variant were evaluated *in silico* with Mutation Taster, Polyphen-2, SIFT, and CADD (see References 35 to 38).

(Induction of Hypertrophy in Cultured Rat Cardiomyoblasts H9c2)

The rat cardiomyoblast cell line H9c2 was obtained from American Type Culture Collection (ATCC; CRL-1466). H9c2 cells were cultured in Dulbecco's modified Eagle medium (DMEM) (Thermo Fisher Scientific, Waltham, MA, USA) supplemented with 10% fetal bovine serum (FBS), 100 units/mL penicillin, and 100 $\mu$g/mL streptomycin. To effectively induce hypertrophy, the H9c2 cells were differentiated with all-trans retinoic acid (ATRA) (Wako Pure Chemical Industries, Osaka, Japan) before induction of hypertrophy (see References 39 and 40). The H9c2 cells were cultured in DMEM containing 1% FBS and 100 mM ATRA for 5 days for differentiation, followed by change to DMEM containing 1% FBS. Hypertrophy was induced by adding 100 nM human endothelin-1 (Sigma-Aldrich, Saint Louis, MO, USA) mixed with mouse IL-17D (R&D systems, Minneapolis, MN, USA). After 24-hour incubation, RNAs were purified from the cells with NucleoSpin RNA isolation kit (Takara Bio, Macherey-Nagel, Dueren, Germany).

(Induction of Hypertrophy in Cultured Human iPSC-derived Cardiomyocytes)

Human iPSC-derived cardiomyocytes (iCell cardiomyocyte2: Fujifilm Cellular Dynamics Inc., Madison, WI, USA) were cultured at 37°C and 5% $CO_2$ in accordance with a manufacturer's instruction. The cardiomyocytes were cultured for 4 hours in a plating medium on a plate coated with fibronectin, followed by medium change to a maintenance medium. After 48 hours, the culture medium was changed to SWE medium prepared by mixing Cell Maintenance Cocktail B (Thermo Fisher Scientific, Waltham, MA, USA) with Williams E medium (Thermo Fisher Scientific, Waltham, MA, USA). The cells were cultured for 3 days. The cardiomyocytes were incubated for 24 hours with human endothelin-1 (Sigma-Aldrich, St. Louis, MO, USA) or phenylephrine (Sigma-Aldrich, St. Louis, MO, USA) mixed with or without human IL-17D (R&D Systems, Minneapolis, MN, USA), and RNAs were purified from the cells with NucleoSpin RNA isolation kit (Takara Bio, Macherey-Nagel, Dueren, Germany).

(Quantitative RT-PCR)

cDNAs were produced by reverse-transcription from the extracted RNAs with Transcriptor High Fidelity cDNA Synthesis Kit (Roche Diagnostic, Basel, Switzerland) or PrimeScript II 1st strand cDNA Synthesis Kit (Takara Bio, Shiga, Japan). Relative gene expression was quantitatively measured by TaqMan probe chemistry from Applied Biosystems or SYBR Green chemistry by TB Green Premix™ DimerEraser™ (Takara Bio, Shiga, Japan) with Applied Biosystems 7500 (Thermo Fisher Scientific, Waltham, MA, USA) or QuantStudio™ 3 (Thermo Fisher Scientific, Waltham, MA, USA). Expression data was normalized relative to ACTB, $\beta$2M, or GAPDH. Quantification was performed by a CT method using a TaqMan probe or a relative standard curve method using SYBR green. With regard to TaqMan probe ID (Thermo Fischer, Waltham, MA, USA), human NPPB corresponded to Hs00173590_m1, and human $\beta$2M corresponded to Hs00187842_m1. Sequences of primer pairs for real-time PCR with SYBR green are as follows: rat ACTA1: 5'-GCTG TGTTCCCATCCATCATCGT-3' (SEQ ID NO: 20) and 5'-GTCAGGATACCTCGCTTGCT-3' (SEQ ID NO: 21); rat ACTB: 5'-ATCGCTGCGCTCGTCGTC-3' (SEQ ID NO: 22) and 5'-CTTCTGACCCATACCCACCA-3' (SEQ ID NO: 23); and rat MYH7: 5'-GCTGAGACAGAATGCAA-3' (SEQ ID NO: 24) and 5'-CCTGATCTTGTCGAACTTG-3' (SEQ ID NO: 25).

(Production of Cell Line stably expressing IL-17D)

Rat IL-17D cDNA was cloned from rat heart cDNA panel (Clontech, Mountain View, CA, USA) by PCR with the following primer pair: IL-rat-cDNA-F, ATGTTGGGACTGGTCTGGATGCCTCG (SEQ ID NO: 26) and IL-rat-cDNA-R, T

CAGCGCCGCCGCCGCCTGTCA (SEQ ID NO: 27).

The cloned rat IL-17D cDNA was inserted into pF5A CMV-neo Flexi Vector (Promega, Madison, WI, USA). The vector thus obtained was transfected in H9c2 cells, rat cardiomyoblasts, and cell lines with Lipofectamine 3000 (Thermo Fisher Scientific, Waltham, MA, USA). The transfected cells were selected with neomycin to provide a rat IL-17D cell line having stable expression. The cells were cultured in DMEM (Thermo Fisher Scientific, Waltham, MA, USA) containing 1% FBS.

(Immunoblotting for Cell Lines)

The cells were recovered with TBS (a buffer containing 25 mM Tris-HCL, pH 8.0, 150 mM NaCl, and 5 mM EDTA) containing 1% Triton X-100, and a protease inhibitor (Sigma-Aldrich, St. Louis, MO, USA), centrifuged at 14,000 g and 4°C for 10 minutes, and separated into a Triton-insoluble membrane fraction as a pellet and a Triton-soluble cytoplasmic fraction as a supernatant (see Reference 41). Equal amounts of the pellet and the supernatant were subjected to SDS-PAGE with 15% polyacrylamide gel, and transferred onto a PVDF membrane (Thermo Fisher Scientific, Waltham, MA, USA). The membrane was blocked with 5% non-fat dry milk, and incubated with an anti-IL-17D rabbit polyclonal antibody (1:200; Santa Cruz, Dallas, TX, USA) at 4°C overnight. After washing with TBS containing 0.05% Tween 20, the membrane was incubated with a secondary goat anti-rabbit IgG horseradish peroxidase (HRP)-labeled antibody (1:500; Dako, Agilent, Santa Clara, CA, USA) for 1 hour.

(Production and Analysis of Cell Line having IL17D Mutant)

H9c2 cells having an IL17D mutation were produced with Guide-it CRISPR-Cas9 system (Takara Bio, Clontech, Mountain View, CA, USA). After hybridization of a pair of target oligos of a gene editing gRNA for the stop codon of rat IL17D (rIL-Guide-F: ccggCAGTCTCATCAGCGCCGC (SEQ ID NO: 28), rIL-Guide-R: aaacGCGGCGCTGATGAGACTG (SEQ ID NO: 29)) was hybridized, and then cloned into pGuide-IT-ZsGreen1 vector having a Cas9 sequence. The pGuide-it-ZsGreen1 vector having target gRNA was transfected into the H9c2 cells with Lipofectamine 3000 reagent (Thermo Fisher Scientific, CA, USA). After 48 hours, cells expressing ZsGreen were selected with MoFlo XDP (Beckman Coulter, Brea, CA, USA), and cultured as single cells. Each single cell clone was cultured, and subjected to Sanger sequencing to verify gene editing (sequencing primers: guide IL-RT1-FN AAGCCTACTGCCTGCCGA (SEQ ID NO: 30), and guide IL-RT1-RN TGGATGGATCGGTGTGCTT (SEQ ID NO: 31)). H9c2 cells having an IL17D mutant or wild-type were cultured at 37°C and 5% $CO_2$ for 24 hours in Dulbecco's modified Eagle medium (Thermo Fisher Scientific, CA, USA) supplemented with 10% fetal bovine serum (FBS) and 1% penicillin/streptomycin, in a state of being mixed with or not containing recombinant mouse IL-17D (R&D Systems, Minneapolis, MN, USA). To differentiate the H9c2 cells, the cells were allowed to reach 70% to 80% confluency and then cultured for 1 week in a medium supplemented with 1% FBS and 100 nM ATRA (Sigma-Aldrich, Saint Louis, MO, USA). Subsequently, RNAs were purified from the cells with NucleoSpin RNA isolation kit (Takara Bio, Macherey-Nagel, Dueren, Germany).

(Genome Editing for IL17D of Human iPSC-derived Cardiomyocytes)

In genome editing with Guide-it CRISPR-Cas9 system (Clontech, Mountain view, CA, USA), target oligos of guide RNAs for gene editing for human IL17D (hIL-KO1-GF: ccggGGAGCAGCTGTACGGCGCC (SEQ ID NO: 32), hIL-KO1-GR: aaacGGCCCCGTACAGCTGCTCC (SEQ ID NO: 33), hIL-KO2-GF: ccggCCTGCGAGCGTGTCGCCCT (SEQ ID NO: 34), hIL-KO2-GR: aaacAGGCGACACGCTGCGCAGG (SEQ ID NO: 35), hIL-KO4-GF: ccggCAACTCCAGCA TCGACAAAC (SEQ ID NO: 36), and hIL-KO4-GR: aaacGTTTGTCGATGCTGGAGTTG (SEQ ID NO: 37)) were cloned into pGuide-it-ZsGreen1 vector. With regard to the guide RNAs, combinations of oligos were separately made as follows: hIL-KO1-GF and hIL-KO1-GR for exon 1 as hG-1; hIL-KO2-GF and hIL-KO2-GR for exon 1 as hG-2; and hIL-KO4-GF and hIL-KO4-GR for exon 2 as hG-3 (Fig. 3A). Human iPSC-derived cardiomyocytes (iCell cardiomyocyte2: Fujifilm, Cellular Dynamics Inc., Madison, WI, USA) were cultured for 6 days in SWE solution on a plate coated with fibronectin. Next, the human iPSC-derived cardiomyocytes were transfected with the pGuide-it-ZsGreen1 vector having each target of the gRNA sequences hG-1, hG-2, and hG-3 with ViaFect transfection reagent (Promega, Madison, WI, USA) over 16 hours. The medium was changed to SWE solution, followed by treatment for 24 hours in SWE solution mixed with or not containing human IL-17D (R&D Systems, Minneapolis, MN, SA). From the human iPS-derived cardiomyocytes thus treated, total RNAs were isolated with NucleoSpin RNA isolation kit (Takara Bio, Macherey-Nagel, Dueren, Germany).

(Investigation of Differentiated Human iPSC-derived Cardiomyocytes)

Human iPSC-derived cardiomyocytes (iCell cardiomyocyte2: Fujifilm-Wako-Cellular Dynamics Inc.) were cultured for 4 hours in a plating medium on a plate coated with fibronectin. Then, 48 hours after change to a maintenance medium, the culture medium was changed to SWE medium. Seven days after the start of culturing of the cells, IL-17D was added so that its concentration was 10 fg/mL or 10 pg/mL. Change to SWE medium supplemented with the same concentration of IL-17D was performed every two days, and after 16 days, RNA extraction was performed with NucleoSpin RNA isolation kit (Takara Bio, Macherey-Nagel, Dueren, Germany).

(RNA-seq Analysis of Human iPSC-derived Cardiomyocytes and Mouse Myocardia)

RNA-seq was performed with RNAs extracted from human iPSC-derived cardiomyocytes (three for each experiment in Fig. 3D, and one for each in Fig. 5D) and mouse myocardia (wild-type and heterozygous mutation of IL17D, three for each). Produce of RNA sequence library, sequencing, and raw data processing were performed in Takara Bio Inc. (Shiga, Japan). The RNA sequence library was produced with SMART-seq v4 Ultra Low Input RNA (Takara Bio, Shiga, Japan). Adaptor sequences were added onto both ends of single-stranded cDNA by Switching Mechanism At 5' end of RNA Template (SMART). PCR amplification was performed with primers that recognized the adaptor sequences. The PCR product thus obtained was purified with AMPure XP (Beckman Coulter, Brea, CA, USA), and used as a double-stranded cDNA product. The double-stranded cDNAs were fragmented by a transposon tagging reaction, and adaptor sequences were added onto both ends. PCR was performed with primers having indexes that recognized adaptor sequences and having tag sequences different among samples. Thus, a sequence library was produced. Pair-end sequencing analysis for 150 bases in length was performed with Nova Seq 6000 (Illumina, San Diego, CA, USA), and output as FASTQ files. Trimming was performed by eliminating adaptors and low-quality sequences from a raw read FASTQ file with CLC genomics Workbench ver. 20 (QIAGEN Aarhus A/S, Aarhus, Denmark). Alignment was performed for human iPSCs on the basis of hg19, and for mouse samples on the basis of mm10. Gene expression was normalized by Transcripts per million (TPM). Each statistical differential expression test on a series of expression tracks was performed by using the differential expression test of the RNA-seq tool of CLC genomics Workbench ver. 20, with use of multiple-factor statistics based on negative binomial GLM. Gene Ontology and functional enrichment analysis for a gene with $P < 0.05$ were performed with Metascape (see Reference 42), and visualized with Prism 9 (GraphPad Software, San Diego, CA, USA).

(Analysis of iPSC-derived Cardiomyocytes with Optical Microscope)

Human iPSC-derived cardiomyocytes (iCell cardiomyocyte2: Fujifilm-Wako-Cellular Dynamics Inc.) were cultured on Chamber slide II coated with fibronectin. The cells were fixed with PBS containing 4% paraformaldehyde (PFA), and treated with PBSAT solution (PBS containing 0.5% Triton X and 1% BSA). The cells were incubated at 4°C overnight with anti-NT-proBNP mouse monoclonal antibody (1:500; Abcam, Cambridge, UK), anti-α-actinin monoclonal antibody (1:800; Abcam, Cambridge, UK), or anti-IL-17D rabbit polyclonal antibody (1:50; Santa Cruz Biotechnology, Dallas, TX, USA) in PBSAT. After washing with PBS, incubation was performed in PBSAT with Alexa568-labeled anti-mouse goat antibody (1:400; Thermo Fisher Scientific, Waltham, MA, USA) for the anti-NT-proBNP antibody, with Alexa488-labeled anti-mouse goat antibody (1:400; Thermo Fisher Scientific, Waltham, MA, USA) for the anti-α-actinin antibody, and with Alexa568-labeled anti-rabbit goat antibody (1:1,000; Thermo Fisher Scientific, Waltham, MA, USA) for the anti-IL-17D antibody. Subsequently, embedding was performed with DAPI-containing Vectashield mounting medium (Vector Laboratory, Newark, CA, USA). Then, the cells were observed with TCS SP8 con-focal laser microscope (Leica Microsystems, Wetzlar, Germany).

(Electron Microscopic Analysis for iPS-derived Cardiomyocytes)

Human iPS-derived cardiomyocytes (iCell cardiomyocyte2: Fujifilm Cellular Dynamics, Inc., Madison, WI, USA) were cultured on Cell Desk LF (Sumitomo Bakelite, Tokyo, Japan) coated with fibronectin. The cells were fixed with 2% PFA and 2% glutaraldehyde (GA) in PBS (pH 7.4). After washing with 0.15 M sodium cacodylate buffer (CB) (pH 7.4), the cells were fixed with 2% osmium tetroxide (OsO4) and 0.8% potassium ferrocyanide in CB, and stained with 2% uranyl acetate (UA) in water. The cells were dehydrated with a series of ethanol solutions, and embedded with Durcupan ACM (Sigma-Aldrich, St. Louis, MO, USA). From the embedded block, 80 nm slices were made. The slices were imaged with H-7100 (Hitachi High-Technologies, Tokyo, Japan) mounted with XR-81 charge-coupled device camera (ATM Imaging system, Woburn, MA, USA).

(Generation and Rearing of Mice having IL17D Variant)

Mice with the stop codon of IL17D gene being skipped were generated with CRISPR-Cas9 gene editing system as described in Reference 13. The sequences of crRNAs having target gRNA and tracrRNA are as follows (with an IL17D-specific sequence underlined): IL-crRNA: 5'-CCGGCAUCAGCGCCGCGUUUAGAGCUAUGCUGUUUG-3' (SEQ ID NO: 38), tracrRNA: 5'-AAACAGCAUAGCAAGUUAAAAAGGCUAGUCCGUUACAACUGAAAAGGCACCGAGUCGUG CU-3' (SEQ ID NO: 39) (Fig. 4A). crRNA, tracrRNA, and Cas9 protein (New England Biolabs, Ipswich, MA, USA) were injected into a fertilized egg, and gene editing was performed. Oligo RNAs were chemically synthesized and purified by high-pressure liquid chromatography (FASMAC, Kanagawa). For genotyping, genomic DNA of each mouse was purified from the tail or an ear piece of a baby mouse with NucleoSpin DNA Rapid Lyse (Takara Bio, Macherey-Nagel, Dueren, Germany). PCR was performed with the following primer pair: mice-IL-F: 5'-AGGAGGACTTCCGCTTTCGC-3' (SEQ ID NO: 40) and mice-IL-R: 5'-AGGAGGACTTCCGCTTTCGC-3' (SEQ ID NO: 41). Genotyping was performed by using Sanger sequencing. A mouse carrying a read-through mutant with the stop codon of IL17D being skipped was selected, and crossed with a wild-type C57BL6 mouse. In this experiment, mice via at least the fifth generation or more from a progenitor were used. Wild-type mice used in Examples are mice of the same generation as that crossed with a mouse carrying an IL17D mutation. Animal experiments were executed under approval by the institutional animal management committees of Tokyo Medical and Dental University, Keio University, and Tokai University.

(Echocardiographic analysis of Mice)

Anesthesia for male mice was started with 5% isoflurane and maintained with 2% isoflurane. Echocardiographic analysis was performed blindly for five wild-type mice and mice having a heterozygous mutation with Vevo 3100 (Fujifilm VisualSonics, Toronto, Canada) by Primetech Inc (Tokyo, Japan). End-diastolic (LVDd) and end-systolic (LVDs) left ventricular diameters and wall thicknesses were measured, and a left ventricular contractile force was evaluated as % fractional shortening

$$(FS) = [(LVDd - LVDs)/LVDd] \times 100.$$

(Sample Preparation and Analysis for Stained Optical Microscopic Images of Mice)

An adult male mouse was euthanized, and subjected to fixation by perfusing PBS containing 4% PFA from the left ventricle at a hydraulic pressure of 90 cm for 5 minutes. The heart was isolated and post-fixed over 4 hours, substituted with 70% ethanol, and then embedded in paraffin. The sample was sliced and stained with HE and Picrosirius red. Each stained sample was observed and imaged with a biological microscope BX63 (Olympus, Tokyo, Japan).

(Sample Preparation and Electron Microscopic Analysis of Mice)

An adult male mouse was euthanized, and the left ventricle underwent perfusion fixation with PBS (pH 7.4) containing 2% PFA and 2% GA at a hydraulic pressure of 90 cm over 5 minutes. The left ventricle was isolated, and a plurality of tissue blocks with a size of 1 mm$^3$ were obtained from the central free wall. The block was post-fixed with the same fixative at 4°C over 2 hours. The fixed tissue was further incubated at 4°C overnight in PBS containing 0.8% potassium ferrocyanide and 2% OsO4. The tissue was stained with 1% uranyl acetate, dehydrated with a series of ethanol solutions, and embedded with Durcupan ACM (Sigma-Aldrich, St. Louis, MO, USA) in accordance with modification of the method described in Reference 44. From the embedded block, 80 nm slices were made. Through use of an electron microscope JEM-1400 (JEOL Ltd., Tokyo, Japan), imaging was performed at a magnification of 10,000× with a 1K×1K pixel camera, and 9 images were tiled to provide an image with 3K×3K pixels in the imaging. For three each of 12-week-old wild-type mice and mutant mice, 10 longitudinal electron microscopic photographs were taken for one mouse. Through use of Fiji/ImageJ, intermyofibrillar mitochondria were traced, the area of the individual mitochondrion was measured, and the occupancy of mitochondrial areas was determined from the area of the whole image (see Reference 45).

(Statistical Analysis)

Statistical analysis was performed by an independent Student t-test with JMP ver. 13 (SAS Institute Inc., Cary, NC, USA) or Prism 9 (GraphPad Software, San Diego, CA, USA). Statistical significance levels were indicated as *p<0.05, **p<0.01, and ***p<0.001 in all figures.

(Example 1) Rare Mutation of IL17D Gene linked to Familial HCM Patient

Whole exome sequencing was performed for familial HCM patients and for unaffected people aged 40 or more, and the heterozygous missense variant of IL17D (NM_138284.1): c.609 A>T, p.Ter203CysextTer51 was identified in all the HCM patients in the family (Fig. 1A and Fig. 1B). IL17D Ter203CysextTer51 was a mutation that was not registered in any of: 1000 genomes project (TG), a variant database for a general population; gnomAD, a variant database for 120,000 or more people; and a human genetic variation database (HGVD) used as a variant database for Japanese people.

While normal IL-17D protein consists of 202 amino acids, IL17D p.Ter203CysextTer51 consists of 253 amino acids (Fig. 1C). Further, 573 genetically unrelated HCM patients who had no cardiomyopathy-related mutation in eight sarcomere genes (MYH7, MYBPC3, TNNT2, TNNI3, MYL3, MYL2, TPM1, and ACTC1) were subjected to gene analysis for a rare IL17D mutation by Sanger method. In sporadic HCM patients, five types of rare IL17D mutants p.Gly22Ser, p.Tyr43His, p.Glu64Val, p.Pro72Leu, and p.Lys175Arg were identified (Fig. 1D). Each proband carried a rare IL17D variant, but carried no etiologic variant of 67 cardiomyopathy-related genes. Each variant was located in a codon of an evolutionarily conserved amino acid (Fig. 1E). Evaluation of mutations at the amino acid coding site of IL17D by a plurality of *in silico* analyses revealed that mutations other than Gly22Ser, i.e., Tyr43His, Glu64Val, Pro72Leu, and Lys175Arg were predictive for functional changes (Table 1).

Table 1

| IL17D variant | Gly22Ser | Tyr43His | Glu64Val | Pro72Leu | Lys175Arg | Ter203Cys extTer51 |
|---|---|---|---|---|---|---|
| Location in genome (hg19) | Chr13: 21278204 G>A | Chr13: 21278267 T>C | Chr13: 21278331 A>T | Chr13: 21278355 C>T | Chr13: 21296008 A>G | Chr13: 21296093 A>T |
| cDNA | c. 64 G>A | c.127 T>C | c.191 A>T | c.215 C>T | c.524 A>G | c.609 A>T |
| Zygosity | Heterozygous | Heterozygous | Heterozygous | Heterozygous | Heterozygous | Heterozygous |
| 1000 genome | none | none | none | none | none | none |
| ExAc | none | none | none | none | none | none |
| gnomAD | none | none | none | 0.000018 | none | none |
| HGVD | none | none | none | none | none | none |
| rs number | none | rs1387984 745 | none | rs1995710 28 | rs1156605 538 | none |
| Mutation Taster | Polymorphism | Disease Causing | Disease Causing | Disease Causing | Disease Causing | NA |
| Polyphen-2 | Benign 0.028 | Probably Damaging 0.976 | Possibly Damaging 0.454 | Probably Damaging 0.999 | Possibly Damaging 0.640 | NA |
| SIFT | Tolerated 0.419 | Damaging 0.004 | Damaging 0.039 | Damaging 0.010 | Tolerated 0.194 | NA |
| CADD | 9.972 | 26 | 23.1 | 26.6 | 22.2 | NA |

Rare variants of IL17D gene found in HCM are shown in Table 1. More specifically, Table 1 shows the allele frequency of each variant in variant databases for a general population 1000 genome, ExAC, gnomAD, and HGVD. As can be seen, those variants are extremely rare. In addition, *in silico* analyses with Mutation Taster, Polyphen-2, SIFT, and CADD were used to predict functional changes in proteins related to the variants. Consequently, functional changes related to the mutations other than Gly22Ser were predicted (shown with predicted function changes double-underlined and "NA" indicating not applicable).

However, there needs further elucidation for a function of IL-17D, particularly a function in myocardia and causal connection with HCM. The inventors of the present invention assumed that IL-17D was a novel myocardial hypertrophy suppressor, and that a pathologic variant of IL17D gene caused a reduction in function of IL-17D, leading to cardiac hypertrophy.

(Example 2) IL-17D improves Drug-induced Cardiac Hypertrophy

To examine a function of IL-17D in cardiac hypertrophy, the inventors of the present invention used drug-induced cardiac hypertrophy models in different cardiomyocyte lines.

First, the rat cardiomyocyte H9c2 cell line differentiated with ATRA was treated with endothelin-1 (ET-1). Thus, myocardial hypertrophy was induced. In addition, an increase in RNA expression level of ACTA1, which encoded skeletal muscle actin, a cardiac hypertrophy marker, was observed (Fig. 2A). Then, it was revealed that, when administered IL-17D in addition to ET-1 and cultured, the differentiated rat cardiomyocytes exhibited improved expression of ACTA1 (Fig. 2A).

In addition, in human iPS cell-derived cardiomyocytes, the RNA expression level of NPPB, a hypertrophy marker, was observed to be increased by ET-1 treatment (Fig. 2B). Further, when cultured with 10 nM ET-1, the human iPS cell-derived cardiomyocytes exhibited strong expression of NT-proBNP, a hypertrophy marker, under optical microscopy (Fig. 2C), and had an increased number of mitochondria under electron microscopy (Fig. 2D), as seen in the findings in human cardiac hypertrophy. In contrast, it was revealed that, when treated with a mixture of 10 nM ET-1 and 0.1 ng/mL or 25 ng/mL recombinant human IL-17D, the human iPS cell-derived cardiomyocytes had suppressed expression of NPPB (Fig. 2B).

It was also observed that, when administered phenylephrine (PE), which induced myocardial hypertrophy in the same manner, at a concentration of 10 $\mu$M, human iPS cell-derived cardiomyocytes exhibited increased expression of NPPB. In contrast, when human recombinant IL-17D was administered in the dose range of from 0.0016 ng/mL to 0.4 ng/mL, RNA expression of NPPB was suppressed (Fig. 2E).

In this way, recombinant IL-17D reduced increases in levels of cardiac hypertrophy markers that were induced by a plurality of drugs in human- and rat-derived cultured cardiomyocytes. IL-17D is conceived to be secreted from cardiomyocytes as well in the same manner as other interleukins. The H9c2 cell line, normal rat cardiomyoblasts, has weak expression of IL-17D. In view of the foregoing, H9c2 cells that stably expressed rat IL-17D were produced, and consequently, high expressions at about 20 kDa and about 50 kDa were detected in the cytoplasmic fraction and the membrane fraction, respectively (Fig. 2F). Those facts suggested that IL-17D was present as a monomer in the cytoplasmic fraction and as a dimer in the cell membrane fraction. In an extracellular medium, IL-17D appeared to be present as a dimer with 50 kDa or more (Fig. 2F). Immunostaining of the differentiated human iPS cell-derived cardiomyocytes with an IL-17D antibody demonstrated that human IL-17D protein tended to be mainly expressed in the nuclei rather than the cytoplasm (Fig. 2G).

(Example 3) Genome Editing of IL17D Gene increases Cardiac Hypertrophy Markers that are recovered by Administration of IL-17D

Human iPS cell-derived cardiomyocytes underwent genome editing of IL17D gene with guide RNAs targeting Exon1 (hG-1 or hG-2) or Exon2 (hG-3) (Fig. 3A). The results demonstrated an increase in expression of NPPB, a marker of cardiac hypertrophy, in association with a decrease in IL-17D function (Fig. 3B). Further, it was revealed that the genome editing in Exon1 (hG-1) of IL17D induced cardiac hypertrophy, and that administration of recombinant human IL-17D was able to rescue increased NPPB at a relatively low concentration (Fig. 3C).

In addition, after the editing of IL17D gene by hG-1 or hG-2 targeting Exon1, the human iPS cell-derived cardiomyocytes were subjected to RNA sequence analysis, and the results showed that cardiac hypertrophy markers (ACTA1 and NPPB), MAPK-related genes characterized by cardiac hypertrophy (MYC and FOS), and several oncogenes increased in both the cells (Fig. 3D). Those expressions were reduced by administration of 10 fg/mL or 1 ng/mL IL-17D (Fig. 3D). In contrast, a cell cycle-related gene cluster (e.g., MCM2 and BRCA2) exhibited reduced expression by the genome editing of IL17D. Those expressions were further downregulated by the addition of IL-17D (Fig. 3D). Those gene expression changes by the gene editing of IL-17D were similar to expression changes by the addition of 10 ng/mL endothelin, which induced cardiac hypertrophy (Fig. 3D).

In addition, ontology analysis of RNA sequences for the cardiomyocytes subjected to the genome editing by hG-1 or hG-2 revealed that genes related to cell death and differentiation, p53 signal transduction, MAPK signal transduction, and HCM increased characteristically (Fig. 3E). In contrast, reduced genes were genes related to cell cycle and DNA replication (Fig. 3F).

(Example 4) Mouse with Read-through Mutation of IL17D exhibits Elevations in Levels of Cardiac Hypertrophy Markers and Increase in Number of Mitochondria

Mice IL17D p.Pro198Alafs*14 with the stop codon of IL17D gene being skipped were produced by gene editing with target guide RNAs (Fig. 4A, Fig. 4B, and Fig. 4C). A 6-month-old IL17D p.Pro198Alafs*14 heterozygous mouse displayed tendencies of cardiac hypertrophy and fibrogenesis as compared to a wild-type mouse (Fig. 4D).

A mouse carrying the IL17D p.Pro198Alafs*14 mutation (11-week-old) was subjected to echocardiographic examination, and consequently found to have an end-systolic left ventricular diameter (LVDs) smaller than that of a wild-type, as shown in Fig. 4E and Fig. 4F. In addition, a left ventricle wall thickness (LVWT) was larger in average in mutant mice, but was found to exhibit no significant difference in this echocardiographic examination in which five mice per group were compared.

Left ventricle myocardia of 12-week-old mice were observed with an electron microscope, and the results showed that mitochondria occupied a wide area within myocardia, and tended to increase and had a small size as compared to those in wild-type mice, as shown in Fig. 4G and Fig. 4H.

RNA-seq analysis for the left ventricle myocardial tissue of a 12-week-old mouse demonstrated that expressions of cardiac hypertrophy markers, such as ACTA1 and NPPB, and mitochondria-related genes, such as MRP and COX, were increased as compared to those of a wild-type mouse. In contrast, expressions of genes related to development and differentiation, such as NOTCH1, AKT, and TBx, were decreased (Fig. 4I). In Gene ontology analysis, expressions of genes related to myocardia and mitochondria typified by muscle system process, TCA cycle, and mitochondrial respiratory chain complex assembly were increased (Fig. 4J), and genes related to development and maturation were decreased (Fig. 4K).

(Example 5) IL-17D promotes Differentiation of Myocardia

Involvement of IL-17D in myocardial differentiation was investigated on the basis of the results of RNA sequencing for human iPS cell-derived cardiomyocytes and mouse myocardia having a read-through mutant of IL17D.

It was revealed that H9c2, an undifferentiated cardiomyoblast, exhibited increased expression of MYH7 by administration of ATRA, which promoted differentiation, and also exhibited increased expression of MYH7 by administration of IL-17D (Fig. 5A).

It was also revealed that H9c2 cells carrying the heterozygous read-through mutation IL17D p.Gly204Alafs*76 produced by genome editing (Fig. 5B) had reduced expression of MYH7, but recovered the expression to a normal level by administration of IL-17D (Fig. 5C). In contrast, H9c2 cells carrying the read-through mutant of IL17D did not exhibit increased MYH7 expression even by treatment with a standard dose of ATRA (Fig. 5C).

Further, RNA sequencing was performed for human iPS cell-derived cardiomyocytes cultured under normal conditions and cells treated with 10 fg/mL or 10 pg/mL IL-17D, and consequently showed that the IL-17D-treated cells exhibited reduced expressions of NPPA and NPPB, natriuretic peptide-related genes whose expressions were increased during cardiac hypertrophy, as shown in Fig. 5D. In contrast, expressions of genes playing functionally important roles in myocardia, such as SLC2A4 encoding GLUT4, ATP2A2 encoding SERCA2, CAV3, RyR2, and SLC8A1 encoding a sodium/calcium exchanger increased expressions of calcium signal-related genes involved in cardiac hypertrophy, insulin, and myocardial excitation-contraction coupling.

Further, with regard to myosin light chain isoforms, MYL2 and MYL3, typically expressed in ventricular myocardia, exhibited no change by the addition of IL-17D, but each gene expression of atrium type MYL4 and MYL7, fetal muscle type MYL5, smooth muscle and non-myocyte type MYL6 and MYL12A was decreased, as shown in Fig. 5E. With regard to potassium channels, the addition of IL-17D increased KCNQ1/J1, expressed in ventricular myocardia, and decreased KCNA5/KCNK3, mainly expressed in the atrium. With regard to sodium channels, the addition of IL-17D provided little change in expression of SCN5A, but increased SCN3B, mainly expressed in the ventricle, and decreased SCN10A, mainly expressed in the atrium. Further, expression of HCN4, a core pacemaker channel, was not changed, but HCN2, mainly expressed in the ventricle, was increased, and HCN1, mainly expressed in the atrium, was decreased.

Those results demonstrated that the administration of IL-17D induced the human iPS cell-derived cardiomyocytes to differentiate to ventricular myocardia.

(Genome Editing for MYBPC3 Gene in Human iPSC-derived Cardiomyocytes)

In genome editing with Guide-it CRISPR-Cas9 system (Clontech, Mountain view, CA, USA), target oligos of guide RNAs for gene editing of human MYBPC3 (hMYBPC3-C0-GF: ccggCAATGAC TGCGTAAGATCCC (SEQ ID NO: 42), hMYBPC3-C0-GR: aaac GGGATCTTACGCAGTCATTG (SEQ ID NO: 43), hMYBPC3-C4-GF: ccgg TGCACACTCA CCGCCCGATG (SEQ ID NO: 44), and hMYBPC3-C4-GR: aaac CATCGGGCGGTGAGTGTGCA (SEQ ID NO: 45)) were cloned into pGuide-it-ZsGreen1 vector.

With regard to guide RNAs, combinations of oligos were separately made as follows: hMYBPC3-C0-GF and hMYBPC3-C0-GR for C0 domain of human MYBPC3 as hG-C0, hMYBPC3-C4-GF and hMYBPC3-C4-GR for C4 domain of human MYBPC3 as hG-C4, and the oligos were hybridized and cloned into pGuide-it-ZsGreen1 vector. Human iPSC-derived cardiomyocytes (iCell cardiomyocyte2: Fujifilm Cellular Dynamics Inc., Madison, WI, USA) were cultured for 6 days in SWE solution on a plate coated with fibronectin. Next, the human iPSC-derived cardiomyocytes were transfected with hG-C0 or hG-C4, which carried each target of gRNA sequences, over 16 hours with ViaFect transfection reagent (Promega, Madison, WI, USA). The medium was changed to SWE solution, and treatment was performed for 24 hours in SWE solution mixed with or not containing human IL-17D (R&D Systems, Minneapolis, MN, SA). From the human iPS-derived cardiomyocytes thus treated, Total RNAs were purified and extracted with NucleoSpin RNA isolation kit (Takara Bio, Macherey-Nagel, Dueren, Germany).

The results are shown in Fig. 6. As shown in Fig. 6, genome editing of MYBPC3, which is the most frequent causative gene for hypertrophic cardiomyopathy, increases the level of a cardiac hypertrophy marker that is then recovered by IL-17D.

The most frequent cause of HCM at home and abroad is MYBPC3 gene, which encodes Cardiac Myosin-binding protein C, and a HCM mutation thereof often generates as a deletion mutation (References 5 and 46). Cardiac Myosin-binding protein C has C0 to C10 domain structures (Reference 47).

Here, human iPS cell-derived cardiomyocytes underwent genome editing of MYBPC3 gene with guide RNAs targeting C0 domain and C4 domain. As a result, as in common HCM, enhanced expression of NPPB gene, a cardiac hypertrophy marker, was observed (C0 and C4 in the supplemental drawing). When IL-17D was further added, expression of NPPB gene was decreased (C0 IL and C4 IL in the supplemental drawing). Those results revealed that IL-17D also had a potential therapeutic effect on common HCM.

Effects of IL-17D on HCM-modeled Mouse Model

With reference to Reference 48, mice carrying mutations in C0 domain and C4 domain of Cardiac Myosin-binding protein C were uniquely produced by genome editing, and observed to display the same phenotype of cardiac hypertrophy as that of HCM. To a HCM-modeled mouse model, IL-17D is administered by subcutaneous injection, intraperitoneal administration, or transvenous administration. The administration of IL-17D can be expected to suppress cardiac hypertrophy.

(References)

1. E. Braunwald, Cardiomyopathies: An Overview. Circ Res 121, 711-721 (2017).
2. W.J. McKenna, B.J. Maron, G. Thiene, Circ Res 121, 722-730 (2017).
3. B.J. Maron et al., Circulation 92, 785-789 (1995).
4. B.J. Maron, M.S. Maron, C. Semsarian, Journal of the American College of Cardiology 60, 705-715 (2012).
5. A.J. Marian, E. Braunwald, Circ Res 121, 749-770 (2017).
6. C.E. Seidman, J.G. Seidman, Circ Res 108, 743-750 (2011).
7. A. Auton et al., Nature 526, 68-74 (2015).
8. K.J. Karczewski et al., Nature 581, 434-443 (2020).
9. K. Higasa et al., Journal of human genetics 61, 547-553 (2016).
10. T. Hayashi et al., Journal of human genetics 63, 989-996 (2018).
11. N. Inagaki et al., Journal of human genetics 63, 1273-1276 (2018).
12. Y.H. Sitbon et al., Journal of muscle research and cell motility 41, 313-327 (2020).
13. C. Collins et al., Human molecular genetics 1, 727-733 (1992).
14. P.S. Bora et al., Genomics 19, 186-188 (1994).
15. I. Park et al., Biochem J 434, 171-180 (2011).
16. Q. Wang et al., Nature genetics 12, 17-23 (1996).
17. D.B. Foster et al., Circ Res 111, 446-454 (2012).
18. T.M. Olson et al., Human molecular genetics 15, 2185-2191 (2006).
19. M.A. Skarsfeldt et al., Pflugers Arch 468, 643-654 (2016).
20. A.I. Fahmi et al., The Journal of physiology 537, 693-700 (2001).
21. S. Pabel et al., Basic research in cardiology 115, 20 (2020).
22. R. Seifert et al., Proceedings of the National Academy of Sciences of the United States of America 96, 9391-9396 (1999).
23. J. Qu et al., Circ Res 89, E8-14 (2001).
24. N. Li et al., Circ Arrhythm Electrophysiol 8, 1219-1227 (2015).
25. M.J. McGeachy et al., Immunity 50, 892-906 (2019).
26. K.H.G. Mills, Nat Rev Immunol. 23, 38-54 (2022).
27. R. Saddawi-Konefka et al., Cell Rep 16, 2348-2358 (2016).
28. R. Seelige et al., Cytokine 91, 10-12 (2017).
29. T. Starnes et al., J Immunol 169, 642-646 (2002)
30. T.A. Moseleyet al., Cytokine Growth Factor Rev 14, 155-174 (2003).
31. M. Wehrens et al., Cell Rep 39, 110809 (2022).
32. S.R. Singh et al., Circulation. Heart failure 10, e004140 (2017).
33. M. Lapierre-Landryet al., Scientific reports 10, 14955 (2020).
34. M. Lek et al., Nature 536, 285-291 (2016).
35. J.M. Schwarz et al., Nature methods 11, 361-362 (2014).
36. I.A. Adzhubei et al., Nature methods 7, 248-249 (2010).
37. P. Kumar, S. et al., Nature protocols 4, 1073-1081 (2009).
38. M. Kircher et al., Nature genetics 46, 310-315 (2014).
39. D. Sumi et al., Biochemical and biophysical research communications 436, 175-179 (2013).

40. K. Hirayama-Yamada et al., Int Heart J 62, 359-366 (2021).

41. T. Hayashi et al., Biochemical and biophysical research communications 313, 178-184 (2004).

42. Y. Zhou et al., Nature communications 10, 1523 (2019).

43. T. Aida et al., Genome Biol 16, 87 (2015).

44. T. Hayashi et al., Journal of cell science 122, 1005-1013 (2009).

45. J.M. Hollander et al., Heart and circulatory physiology 307, H1-14 (2014).

46. Topriceanu CC et al. Meta-Analysis of Penetrance and Systematic Review on Transition to Disease in Genetic Hypertrophic Cardiomyopathy. Circulation 2024. 149(2): 107-123.

47. Flashman E et al. Cardiac myosin binding protein C: its role in physiology and disease. Circ Res. 2004, 94(10): 1279-89.

48. Vignier N et al. Nonsense-mediated mRNA decay and ubiquitin-proteasome system regulate cardiac myosin-binding protein C mutant levels in cardiomyopathic mice. Circ Res. 2009. 105(3): 239-248.

Industrial Applicability

As has been described so far, it was revealed that IL-17D was a cardiac hypertrophy suppressor and had a function to promote differentiation of cardiomyocytes. Accordingly, in the present invention, the use of IL-17D enables suppression of cardiac hypertrophy, and in turn, treatment of a cardiac disease such as HCM. In addition, examination for cardiac hypertrophy can be performed by using IL-17D as an indicator. Further, the use of cardiomyocytes or the like having a suppressed function of IL-17D enables screening for a substance having activity to suppress cardiac hypertrophy. In addition, the use of IL-17D also enables promotion of differentiation of immature cardiomyocytes.

Accordingly, the present invention is useful in the field of medicine of a cardiac disease associated with cardiac hypertrophy. The present invention is also useful in regenerative medicine with use of differentiated cardiomyocytes, screening for a substance having cardiac toxicity with use of the cells, and the like.

**Claims**

1. A composition for suppressing cardiac hypertrophy, comprising interleukin-17D as an active ingredient.

2. The composition according to claim 1, wherein the pharmaceutical composition is a pharmaceutical composition for treating or preventing a disease associated with cardiac hypertrophy.

3. An examination method for cardiac hypertrophy, comprising detecting interleukin-17D for a sample isolated from a test subject.

4. A screening method for a substance having activity to suppress cardiac hypertrophy, comprising the steps of:

   bringing a cardiomyocyte having a suppressed function of interleukin-17D into contact with a test substance;
   detecting expression of a cardiac hypertrophy marker in the cardiomyocyte; and
   determining the test substance to have activity to suppress cardiac hypertrophy when the expression of the cardiac hypertrophy marker detected in the detecting step is reduced as compared to no contact with the test substance.

5. A screening method for a substance having activity to suppress cardiac hypertrophy, comprising the steps of:

   administering a test substance to a non-human animal having a suppressed function of interleukin-17D;
   detecting cardiac hypertrophy in the non-human animal; and
   determining the test substance to have activity to suppress cardiac hypertrophy when the cardiac hypertrophy detected in the detecting step is reduced as compared to no administration of the test substance.

6. A production method for a differentiated cardiomyocyte, comprising a step of culturing an immature cardiomyocyte in the presence of interleukin-17D.

7. The production method according to claim 6, wherein the immature cardiomyocyte is an immature cardiomyocyte induced to differentiate from a pluripotent stem cell, or a cardiomyoblast.

Fig. 1A

Fig. 1B

Fig. 1C

IL17D: Wild type (202 amino acids)

MLVAGFLLALPPSWAAGAPRAGRRPARPRGCADRPEELLEQLYGRLAAGVLSAFHHTLQLGPRE
QARNASCPAGGRPADRRFRPPTNLRSVSPWAYRISYDPARYPRYLPEAYCLCRGCLTGLFGEEDV
RFRSAPVYMPTVVLRRTPACAGGRSVYTEAYVTIPVGCTCVPEPEKDADSINSSIDKQGAKLLLGPN
DAPAGP *

IL17D p.Ter203CysextTer51 (253 amino acids)

MLVAGFLLALPPSWAAGAPRAGRRPARPRGCADRPEELLEQLYGRLAAGVLSAFHHTLQLGPRE
QARNASCPAGGRPADRRFRPPTNLRSVSPWAYRISYDPARYPRYLPEAYCLCRGCLTGLFGEEDV
RFRSAPVYMPTVVLRRTPACAGGRSVYTEAYVTIPVGCTCVPEPEKDADSINSSIDKQGAKLLLGPN
DAPAGPCGRSCPGRSPRPASRGAQAGAAWRARSATSEESAPSKPSAGAPAPPFHGDS*

Fig. 1D

**Familial**

Ter203Cys
extTer51

**Sporadic**

| Gly22Ser | Tyr43His | Glu64Val | Pro72Leu | | Lys175Arg |

5'                                                                                     3'

Fig. 1E

```
          64
          V
Human      ML------V  AGFLLALPPS  WAAGAPRAGR  EDARNASCFA  RPARPRGCAD  RPEELLEQLY  GRLAAGVLSA  FHHTLQLGPR  63
Orangutan  ML------V  AGFLLALPPG  QAAGAPRAGR  EDARNASCFA  RPARPRGCAD  RPEELLEQLY  GRLAAGVLSA  FHHTLQLGPR  63
Bovine     -V-----WALV AGVLLALSPG  RAAGAPKAGR  EDARNASCFA  RPARARGCAD  RPEELLEQLY  GRLAAGVLGA  FHHTLQLGPR  65
Goat       MV-----WALV AGVLLALSPG  RAAGAPKAGR  EDARNASCFA  RPARPRGCAE  RPEELLEQLY  GRLAAGVLGA  FHHTLQLGPR  66
Cat        ML------A  VGVLLALAPG  RAAGALRASR  EDARNATLFG  A--ARPRDCAD  RPRSSLEQLY  GGWPAGLPAP  SHOHLQLGPR  63
Rat        MLGTLVWMPS  SA-PVALAPG  RAAGAARHGK  EDARNASCFA  RCPPLGPYRI  SYDPARYPKY  LPEAYCLCRG  CLTGLFGEED  68
Mouse      MLGTLVWMLA  VGFLLALAPG  RAAGALRTGR  EDARNASCFA  RPARPRDCAD  RPEELLEQLY  GRLAAGVLSA  FHHTLQLGPR  70
Xenopus    MM------LL  FGILIA----  SCNGSKPVKR  DK-ENISCFA  PPPKPKSCAD  RPEEHLEQVY  GRLAAGMLSA  YHHTLQLQPL  60
Zebrafish  MRAPVVSTLV  LVWLWCCGSA  PGERGQKVSR  ER-QNLSCF  RAPRTRSCLD  LPEEILEOMF  GRLSVGVLSA  FHHTLQLATP  70

          72                        22
          L                         S
Human      VRFRSAPVYM  PTVLRRTPA  -CAGGRSVYT  G--GRPA-DR  RFRPPTNLRS  VSP--WAYRI  LPEAYCLCRG  CLTGLFGEED  128
Orangutan  VRFRSAPVYM  PTVVLRRTPA  -CAGGRSVYT  G--GRPA-DR  RFRPPTNLRS  VSP--WAYRI  LPEAYCLCRG  CLTGLFGEED  128
Bovine     VRLRSAPVLV  PAVVLRRTPG  -CAGGRAVVY  G--GRPA-DR  RFRPPTNLRS  VSP--WAYRI  LPEAYCLCRG  CLTGPRGEED  130
Goat       VRLRSAPVLV  PTVILRRTSA  -CAGGRWVYT  G--GRPA-DR  RFRPPTNLRS  VSP--WAYRI  LPEAYCLCRG  CLTGPRGEED  131
Cat        LRFRSAPVYM  PAVVLRRTAA  -CAGGRSVYA  G--GRPG-DR  RFRPRHHLRS  VSP--WAYRI  LPEAYCLCRG  CLTGLFGEED  128
Rat        FRFRSTPVFS  PAVVLRRTAA  -CAGGRSVVA  R--GRSX-DR  RFRPRAHHLRS VSP--WAYRI  LPEAYCLCRG  CLTGLYGEED  135
Mouse      FRFRSTPVFS  PTVILRRTTS  -CAGGRYVYE  G--GRAA-DR  RFRPPTNLRS  ISP--WAYRI  LPEAYCLCRG  CLTGLYGEED  135
Xenopus    LNFRSMPVYM  PTVILRRTVG  PCVGGRHSYT  GTQGRGAGDG  KQRLPVNIHS  ISP--WAYRI  IPEAYCLCKG  CLGLLGEED  127
Zebrafish  DRFRSTPVYM                         GSASRLARDR  P-RTPVNLLS  LSP--WAYRI  LPEAYCLCKG  CLSGLNGEES  134

          43
          H
Human      EAYVTIPVGC  TCVPEPEKDA  D---SINSSI  DKQGAKLLLG  194
Orangutan  EAYVTIPVGC  TCVPEPEKDA  D---SINSSI  DKQGAKLLLG  194
Bovine     EEYVTVPVGC  TCVPGPEKDA  D---AANSSL  DKS------  189
Goat       EEYVTVPVGC  TCVPGPEKDA  D---AVNSSL  DKS------  190
Cat        EHYITIPVGC  TCVPEQEKEA  D---AVNSSM  DKQGARLLLG  194
Rat        EHYITIPVGC  TCVPEPEKSA  D---MLLG  DK----MLLG  197
Mouse      EEYITIPVGC  TCVPEPEKSA  D---SANSSM  DK----LLLG  197
Xenopus    ESYVSIAVGC  TCVPEQEKGA  ELLESVNSSL  EKEKFKLPLN  196
Zebrafish             TCVPLLEDS  KIQKSKPSVR  RVASKNTVSS  204

          175
          R
Human      PNDAPAGP  202
Orangutan  PNHAPAGP  202
Bovine     -----ARP  192
Goat       -----ARP  193
Cat        PGDKPGRP  202
Rat        VADRPAGR  205
Mouse      PADRPAGR  205
Xenopus    KSEKPLAN  204
Zebrafish  TFRKNELN  212
```

Fig. 2A

Fig. 2B

Fig. 2C

Fig. 2D

Fig. 2E

Fig. 2F

Fig. 2G

Fig. 3A

Fig. 3B

Fig. 3C

Fig. 3D

Fig. 3E

Fig. 3F

Fig. 4A

Fig. 4B

Fig. 4C

**Mouse IL17D :Wild type (205 amino acids)**

MLGTLVWMLAVGFLLALAPGRAAGALRTGRRPARPRDCADRPEEL
LEQLYGRLAAGVLSAFHHTLQLGPREQARNASCPAGGRAADRRFR
PPTNLRSVSPWAYRISYDPARFPRYLPEAYCLCRGCLTGLYGEEDFR
FRSTPVFSPAVVLRRTAACAGGRSVYAEHYITIPVGCTCVPEPDKSA
DSANSSMDKLLLGPADRPAGR*

**Mouse IL17D p.Pro198Alafs*14 (212 amino acids)**

MLGTLVWMLAVGFLLALAPGRAAGALRTGRRPARPRDCADRPEEL
LEQLYGRLAAGVLSAFHHTLQLGPREQARNASCPAGGRAADRRFR
PPTNLRSVSPWAYRISYDPARFPRYLPEAYCLCRGCLTGLYGEEDFR
FRSTPVFSPAVVLRRTAACAGGRSVYAEHYITIPVGCTCVPEPDKSA
DSANSSMDKLLLGARHGPASCMHQVPWP*

Fig. 4D

Fig. 4E

Fig. 4F

Fig. 4G

Fig. 4H

Fig. 4I

log$_2$(fold change)/WT

Fig. 4J

Fig. 4K

Fig. 5A

Fig. 5B

Fig. 5C

Fig. 5D

Fig. 5E

Fig. 6

C0 DOMAIN

C4 DOMAIN

## INTERNATIONAL SEARCH REPORT

| | International application No. |
|---|---|
| | **PCT/JP2024/010348** |

| | |
|---|---|
| **A. CLASSIFICATION OF SUBJECT MATTER** | |

***A61K 38/20***(2006.01)i; ***A61P 9/00***(2006.01)i; ***C07K 14/54***(2006.01)i; ***C12N 5/0735***(2010.01)i; ***C12N 5/077***(2010.01)i; ***C12Q 1/02***(2006.01)i; ***G01N 33/15***(2006.01)i; ***G01N 33/50***(2006.01)i; ***G01N 33/68***(2006.01)i
FI: A61K38/20; A61P9/00; C07K14/54; C12N5/0735; C12N5/077; C12Q1/02; G01N33/15 Z; G01N33/50 Z; G01N33/68

According to International Patent Classification (IPC) or to both national classification and IPC

| | |
|---|---|
| **B. FIELDS SEARCHED** | |

Minimum documentation searched (classification system followed by classification symbols)

A61K38/20; A61P9/00; C07K14/54; C12N5/0735; C12N5/077; C12Q1/02; G01N33/15; G01N33/50; G01N33/68

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

| | |
|---|---|
| **C. DOCUMENTS CONSIDERED TO BE RELEVANT** | |

| Category* | Citation of document, with indication, where appropriate, of the relevant passages | Relevant to claim No. |
|---|---|---|
| X | IDE-IWATA, N. et al. Interleukin 27 inhibits isoproterol-induced cardiac hypertrophy. J Mol Cell Cardiol. 2010, vol. 48, p. S14, ISSN 0022-2828 column P-1-9-3 | 1-2 |
| X | 野出孝一, 89. 心血管不全に対する新規治療法開発の基礎的研究, 上原記念生命科学財団研究報告集, 2019, vol. 33, pp. 1-3, ISSN 2433-3441. (Research reports of The Uehara Memorial Foundation), non-official translation (NODE, Koichi. 89. Fundamental study on development of new treatment for cardiovascular failure.) in particular, Introduction, Results and discussion, etc. | 1-2 |

☐ Further documents are listed in the continuation of Box C.    ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **16 May 2024** | **04 June 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

## INTERNATIONAL SEARCH REPORT

| International application No. |
| --- |
| **PCT/JP2024/010348** |

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT

International application No.

PCT/JP2024/010348

| Box No. III | Observations where unity of invention is lacking (Continuation of item 3 of first sheet) |
|---|---|

This International Searching Authority found multiple inventions in this international application, as follows:

Document 1: IDE-IWATA, N. et al. Interleukin 27 inhibits isoproterol-induced cardiac hypertrophy. J Mol Cell Cardiol. 2010, vol. 48, p. S14, ISSN 0022-2828

Document 2: 野出孝一, 89. 心血管不全に対する新規治療法開発の基礎的研究, 上原記念生命科学財団研究報告集, 2019, vol. 33, pp. 1-3, ISSN 2433-3441, (Research reports of The Uehara Memorial Foundation) in particular, Introduction, Results and discussion, etc., non-official translation (NODE, Koichi. 89. Fundamental study on development of new treatment for cardiovascular failure.)

(Invention 1) Claims 1-2

The invention in claims 1-2 lacks novelty in light of document 1 (column P-1-9-3) and document 2 (in particular, Introduction, Results and discussion, etc.) and thus does not have a special technical feature.

Therefore, the invention in claims 1-2, for which the presence or absence of a special technical feature was previously determined, is classified as invention 1.

(Invention 2) Claim 3

The invention in claim 3 shares only the common technical feature of "Interleukin-17D and cardiac hypertrophy" with the invention classified as invention 1. However, said technical feature does not make a contribution over the prior art in light of the disclosures of documents 1 and 2 and thus cannot be said to be a special technical feature.

Also, it cannot be said that there are no other same or corresponding special technical features between the invention 1 and the invention in claim 3.

In addition, claim 3 is not substantially identical to or similarly closely related to any of the claims classified as invention 1.

Also, claim 3 is not dependent on any of claims classified as invention 1.

Therefore, the invention in claim 3 cannot be classified as invention 1 and is thus classified as invention 2.

(Invention 3) Claims 4 and 5

The invention in claims 4-5 shares only the common technical feature of "Interleukin-17D and cardiac hypertrophy" with the invention classified as any of inventions 1 and 2. However, said technical feature does not make a contribution over the prior art in light of the disclosures of documents 1 and 2 and thus cannot be said to be a special technical feature.

Also, it cannot be said that there are no other same or corresponding special technical features between the inventions 1 and 2 and the invention in claims 4 and 5.

In addition, claims 4 and 5 are not substantially identical to or similarly closely related to any of the claims classified as inventions 1 and 2.

Furthermore, claims 4 and 5 are not dependent on any of claims classified as inventions 1 and 2.

Therefore, the invention in claims 4 and 5 cannot be classified as any of inventions 1 and 2 and is thus classified as invention 3.

(Invention 4) Claims 6 and 7

The invention in claims 6 and 7 shares only the common technical feature of "Interleukin-17D and cardiac hypertrophy" with the invention classified as any of inventions 1-3. However, said technical feature does not make a contribution over the prior art in light of the disclosures of documents 1 and 2 and thus cannot be said to be a special technical feature.

Also, it cannot be said that there are no other same or corresponding special technical features between the inventions 1-3 and the invention in claims 6 and 7.

In addition, claims 6 and 7 are not substantially identical to or similarly closely related to any of the claims classified as inventions 1-3.

Furthermore, claims 6 and 7 are not dependent on any of claims classified as inventions 1-3.

Therefore, the invention in claims 6 and 7 cannot be classified as any of inventions 1-3 and is thus classified as invention 4.

Form PCT/ISA/210 (continuation of first sheet) (July 2022)

## INTERNATIONAL SEARCH REPORT

| International application No. |
| --- |
| **PCT/JP2024/010348** |

**Box No. III    Observations where unity of invention is lacking (Continuation of item 3 of first sheet)**

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **claims 1-2**

**Remark on Protest**    ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013111875 A1
- WO 2015182765 A1

**Non-patent literature cited in the description**

- **B.J. MARON et al.** *Journal of the American College of Cardiology*, 2012, vol. 60, 705-715
- **A.J. MARIAN et al.** *Circ Res*, 2017, vol. 121, 749-770
- **C.E. SEIDMAN et al.** *Circ Res*, 2011, vol. 108, 743-750
- **R. SADDAWI-KONEFKA et al.** *Cell Rep*, 2016, vol. 16, 2348-2358
- **R. SEELIGE et al.** *Cytokine*, 2017, vol. 91, 10-12
- **T. STARNES et al.** *J Immunol*, 2002, vol. 169, 642-646
- **TIMOTHY O'SULLIVAN et al.** *Cell Rep*, 2014, vol. 7 (4), 989-998
- **MINAMI I et al.** *Cell Rep.*, 29 November 2012, vol. 2 (5), 1448-60
- **LIAN, X. et al.** *Nat. Protoc.*, 2013, vol. 8, 162-175
- **E. BRAUNWALD**. Cardiomyopathies: An Overview. *Circ Res*, 2017, vol. 121, 711-721
- **W.J. MCKENNA ; B.J. MARON ; G. THIENE**. *Circ Res*, 2017, vol. 121, 722-730
- **B.J. MARON et al.** *Circulation*, 1995, vol. 92, 785-789
- **B.J. MARON ; M.S. MARON ; C. SEMSARIAN**. *Journal of the American College of Cardiology*, 2012, vol. 60, 705-715
- **A.J. MARIAN ; E. BRAUNWALD**. *Circ Res*, 2017, vol. 121, 749-770
- **C.E. SEIDMAN ; J.G. SEIDMAN**. *Circ Res*, 2011, vol. 108, 743-750
- **A. AUTON et al.** *Nature*, 2015, vol. 526, 68-74
- **K.J. KARCZEWSKI et al.** *Nature*, 2020, vol. 581, 434-443
- **K. HIGASA et al.** *Journal of human genetics*, 2016, vol. 61, 547-553
- **T. HAYASHI et al.** *Journal of human genetics*, 2018, vol. 63, 989-996
- **N. INAGAKI et al.** *Journal of human genetics*, 2018, vol. 63, 1273-1276
- **Y.H. SITBON et al.** *Journal of muscle research and cell motility*, 2020, vol. 41, 313-327
- **C. COLLINS et al.** *Human molecular genetics*, 1992, vol. 1, 727-733
- **P.S. BORA et al.** *Genomics*, 1994, vol. 19, 186-188
- **I. PARK et al.** *Biochem J*, 2011, vol. 434, 171-180
- **Q. WANG et al.** *Nature genetics*, 1996, vol. 12, 17-23
- **D.B. FOSTER et al.** *Circ Res*, 2012, vol. 111, 446-454
- **T.M. OLSON et al.** *Human molecular genetics*, 2006, vol. 15, 2185-2191
- **M.A. SKARSFELDT et al.** *Pflugers Arch*, 2016, vol. 468, 643-654
- **A.I. FAHMI et al.** *The Journal of physiology*, 2001, vol. 537, 693-700
- **S. PABEL et al.** *Basic research in cardiology*, 2020, vol. 115, 20
- **R. SEIFERT et al.** *Proceedings of the National Academy of Sciences of the United States of America*, 1999, vol. 96, 9391-9396
- **J. QU et al.** *Circ Res*, 2001, vol. 89, E8-14
- **N. LI et al.** *Circ Arrhythm Electrophysiol*, 2015, vol. 8, 1219-1227
- **M.J. MCGEACHY et al.** *Immunity*, 2019, vol. 50, 892-906
- **K.H.G. MILLS**. *Nat Rev Immunol.*, 2022, vol. 23, 38-54
- **T.A. MOSELEY ET**. *Cytokine Growth Factor Rev*, 2003, vol. 14, 155-174
- **M. WEHRENS et al.** *Cell Rep*, 2022, vol. 39, 110809
- **S.R. SINGH et al.** Circulation. *Heart failure*, 2017, vol. 10, e004140
- **M. LAPIERRE-LANDRY ET**. *Scientific reports*, 2020, vol. 10, 14955
- **M. LEK et al.** *Nature*, 2016, vol. 536, 285-291
- **J.M. SCHWARZ et al.** *Nature methods*, 2014, vol. 11, 361-362
- **I.A. ADZHUBEI et al.** *Nature methods*, 2010, vol. 7, 248-249
- **P. KUMAR, S. et al.** *Nature protocols*, 2009, vol. 4, 1073-1081
- **M. KIRCHER et al.** *Nature genetics*, 2014, vol. 46, 310-315

- **D. SUMI et al.** *Biochemical and biophysical research communications*, 2013, vol. 436, 175-179
- **K. HIRAYAMA-YAMADA et al.** *Int Heart J*, 2021, vol. 62, 359-366
- **T. HAYASHI et al.** *Biochemical and biophysical research communications*, 2004, vol. 313, 178-184
- **Y. ZHOU et al.** *Nature communications*, 2019, vol. 10, 1523
- **T. AIDA et al.** *Genome Biol*, 2015, vol. 16, 87
- **T. HAYASHI et al.** *Journal of cell science*, 2009, vol. 122, 1005-1013
- **J.M. HOLLANDER et al.** *Heart and circulatory physiology*, 2014, vol. 307, H1-14
- **TOPRICEANU CC et al.** Meta-Analysis of Penetrance and Systematic Review on Transition to Disease in Genetic Hypertrophic Cardiomyopathy. *Circulation*, 2024, vol. 149 (2), 107-123
- **FLASHMAN E et al.** Cardiac myosin binding protein C: its role in physiology and disease. *Circ Res.*, 2004, vol. 94 (10), 1279-89
- **VIGNIER N et al.** Nonsense-mediated mRNA decay and ubiquitin-proteasome system regulate cardiac myosin-binding protein C mutant levels in cardiomyopathic mice. *Circ Res.*, 2009, vol. 105 (3), 239-248